# EUROPÄISCHE PATENTSCHRIFT

(11) **EP 1 383 751 B1**
(45) Veröffentlichungstag und Bekanntmachung des Hinweises auf die Patenterteilung: **17.01.2007**
(21) Anmeldenummer: 02764014.3
(22) Anmeldetag: 10.04.2002
(51) Int. Cl.: C07D 277/20, C07D 417/12, C07D 277/38, A01N 47/40, A01N 51/00

(54) **NEUE INSEKTIZID WIRKENDE AZOLE**
NOVEL AZOLES HAVING AN INSECTICIDAL ACTION
NOUVEAUX AZOLES A ACTION INSECTICIDE

(30) Priorität: 20.04.2001 DE 10119423
(43) Veröffentlichungstag der Anmeldung: 28.01.2004
(73) Patentinhaber: Bayer CropScience AG, 40789 Monheim (DE)
(72) Erfinder: JESCHKE, Peter, 51467 Bergisch Gladbach (DE); BECK, Michael, 41363 Jüchen (DE); KRÄMER, Wolfgang, 51399 Burscheid (DE); WOLLWEBER, Detlef, 42133 Wuppertal (DE); ERDELEN, Christoph, 42799 Leichlingen (DE); TURBERG, Andreas, 42781 Haan (DE); HANSEN, Olaf, 42799 Leichlingen (DE); MARTIN, Hans-Dieter, 40591 Düsseldorf (DE); SAUER, Piet, 89518 Heidenheim (DE)
(86) Internationale Anmeldenummer: PCT/EP2002/003976
(87) Internationale Veröffentlichungsnummer: WO 2002/085870

(56) Entgegenhaltungen:
- EP-A- 0 192 060
- EP-A- 0 483 055
- EP-A- 0 547 557
- EP-A- 0 697 410
- DE-A- 2 921 683

## Beschreibung

Die vorliegende Erfindung betrifft neue heterocyclische Verbindungen, Verfahren zu ihrer Herstellung und ihre Verwendung als Pflanzenschutzmittel, insbesondere zur Bekämpfung von tierischen Schädlingen.

Verschiedene aminosubstituierte Imino-Heterocyclen, wie beispielsweise das 2-Imino-4-amino-thiazolin, sind bereits als Zwischenprodukte für Farb- und Wirkstoffe oder auch als Substanzen mit einem strahlenschützenden Effekt bekannt geworden. Über eine Verwendung als Pflanzenschutzmittel und insbesondere zur Bekämpfung von tierischen Schädlingen ist jedoch nichts bekannt (vgl. DE-OS 2 921 683; S.M. Abdelall et al. J. Heterocyclic Chem. 25, 1849-1956, 1988; E.N. Goncharenko et al. Radiobiologiya 22(2), 252-255, 1982).

Weitere aminosubstituierte Imino-Heterocyclen, wie beispielsweise die 3-Amino- und 3-Alkylamino-5-imino-2,5-dihydrofurane sind bekannt (vgl. S.R Landor J. Chem. Eoc. Perkin Trans. 1201-1204, 1991; M. Yu. Skvortsov et al. Zh. Org. Khim. 27(3), 526-530, 1991; M. Yu. Skvortsov et al. Zh. Org. Khim. 22(2), 255-259, 1986; M. Yu. Skvortsov et al., Zh. Org. Khim. 21(1), 221-222, 1985). Bestimmte Imino-Heterocyclen dieses Strukturtyps, beispielsweise das 2-Imino-4-(2-hydroxyethyl-amino)-5,5-dimethyl-2,5-dihydrofuran, zeigen entzündungshemmende und analgetische Aktivität (vgl. B.A. Trofimov et al. SU 1392872, 1998). Über die Verwendung als Pflanzenschutzmittel, insbesondere zur Bekämpfung von tierischen Schädlingen, ist jedoch nichts bekannt geworden.

EP 0 192 060 beschreibt hererocyclische Verbindungen und ihre Verwendung als insektizides Mittel. Es wurden nun neue heterocyclische Verbindungen der Formel (I) gefunden, 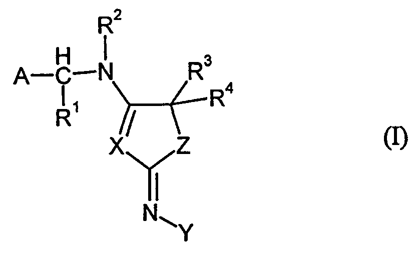 in welcher
- A: für jeweils gegebenenfalls substituiertes Aryl oder Hetaryl der Heterocyclyl steht,
- R¹: für Wasserstoff oder C₁-C₃-Alkyl steht,
- R²: für Wasserstoff oder C₁-C₃-Alkyl steht,
- R³: für Wasserstoff oder C₁-C₃-Alkyl steht,
- R⁴: für Wasserstoff oder C₁-C₃-Alkyl steht,
- X: für N oder CH steht,
- Y: für CN oder NO₂ steht,
- Z: für CH₂, O, S, SO, SO₂ oder NR⁵ steht und
- R⁵: für Wasserstoff oder C₁-C₃-Alkyl steht.

Weiterhin wurde gefunden, dass man die Verbindungen der Formel (I) erhält, wenn man Verbindungen der Formel (II) 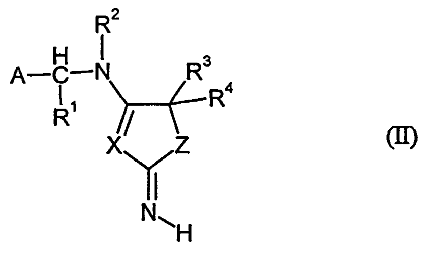 mit geeigneten Cyanierungsreagenzien (zum Erhalt von Verbindungen der Formel (I) in welchen Y für CN steht) oder mit geeigneten Nitrierungsreagenzien (zum Erhalt von Verbindungen der Formel (I) in welchen Y für NO₂ steht) umsetzt.

Schließlich wurde gefunden, dass die neuen Verbindungen der Formel (I) stark ausgeprägte biologische Eigenschaften besitzen und vor allem zur Bekämpfung von tierischen Schädlingen, insbesondere von Insekten, Spinnentieren und Nematoden, die in der Landwirtschaft, in den Forsten, im Vorrats- und Materialschutz sowie auf dem Hygienesektor vorkommen, geeignet sind.

Die erfindungsgemäßen Verbindungen sind durch die Formel (I) allgemein definiert.

Bevorzugte Substituenten bzw. Bereiche der in den oben und nachstehend erwähnten Formeln aufgeführten Reste werden im folgenden erläutert.
- A: steht bevorzugt für gegebenenfalls durch Halogen, Cyano, Nitro, C₁-C₄-Alkyl, C₁-C₄-Halogenalkyl, C₁-C₄-Alkoxy oder C₁-C₄-Halogenalkoxy substituiertes Phenyl.
- A: steht bevorzugt weiterhin für Pyrazolyl, 1,2,4-Triazolyl, Oxazolyl, Isoxazolyl, Thiazolyl, Isothiazolyl, 1,2,5-Thiadiazolyl, Pyridyl, Pyrazinyl oder Pyrimidinyl, welche gegebenenfalls durch Fluor, Chlor, Brom, Cyano, Nitro, C₁-C₂-Alkyl (welches gegebenenfalls durch Fluor und/oder Chlor substituiert ist), C₁-C₂-Alkoxy (welches gegebenenfalls durch Fluor und/oder Chlor substituiert ist), C₁-C₂-Alkylthio (welches gegebenenfalls durch Fluor und/oder Chlor substituiert ist) oder C₁-C₂-Alkylsulfonyl (welches gegebenenfalls durch Fluor und/oder Chlor substituiert ist) substituiert sind.
- A: steht weiterhin für einen gegebenenfalls durch Halogen oder C₁-C₃-Alkyl substituierten gesättigten C₅-C₆-Cycloalkylrest, in welchem eine Methylengruppe durch O oder S ersetzt ist.
- R¹: steht bevorzugt für Wasserstoff, Methyl, Ethyl, n-Propyl oder i-Propyl.
- R²: steht bevorzugt für Wasserstoff, Methyl, Ethyl, n-Propyl oder i-Propyl.
- R³: steht bevorzugt für Wasserstoff, Methyl, Ethyl, n-Propyl oder i-Propyl.
- R⁴: steht bevorzugt für Wasserstoff, Methyl, Ethyl, n-Propyl oder i-Propyl.
- X: steht bevorzugt für N oder CH.
- Y: steht bevorzugt für CN oder NO₂.
- Z: steht bevorzugt für CH₂, O, S oder NR⁵.
- R⁵: steht bevorzugt für Wasserstoff, Methyl, Ethyl, n-Propyl oder i-Propyl.
- A: steht besonders bevorzugt für Thiazolyl oder Pyridyl, welche jeweils gegebenenfalls substituiert sind durch Halogen (insbesondere Chlor) oder C₁-C₃-Alkyl (insbesondere Methyl).
- A: steht weiterhin besonders bevorzugt für einen gegebenenfalls durch Halogen (insbesondere Chlor) oder C₁-C₃-Alkyl (insbesondere Methyl) substituierten Tetrahydrofurylrest.
- R¹: steht besonders bevorzugt für Wasserstoff oder Methyl.
- R²: steht besonders bevorzugt für oder Methyl oder Ethyl.
- R³: steht besonders bevorzugt für Wasserstoff oder Methyl.
- R⁴: steht besonders bevorzugt für Wasserstoff oder Methyl.
- X: steht besonders bevorzugt für N oder CH.
- Y: steht besonders bevorzugt für CN oder NO₂.
- Z: steht besonders bevorzugt für CH₂, O, S oder NR⁵.
- R⁵: steht besonders bevorzugt für Wasserstoff oder Methyl.
- A: steht ganz besonders bevorzugt für einen der Reste

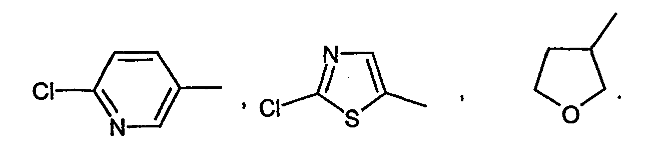

- R¹: steht ganz besonders bevorzugt für Wasserstoff oder Methyl.
- R²: steht ganz besonders bevorzugt für Methyl oder Ethyl.
- R³: steht ganz besonders bevorzugt für Wasserstoff oder Methyl.
- R⁴: steht ganz besonders bevorzugt für Wasserstoff oder Methyl.
- X: steht ganz besonders bevorzugt für N oder CH.
- Y: steht ganz besonders bevorzugt für CN oder NO₂.
- Z: steht ganz besonders bevorzugt für CH₂, O, S oder NR⁵.
- R⁵: steht ganz besonders bevorzugt für Wasserstoff oder Methyl.

In einer hervorgehobenen Gruppe von Verbindungen der Formel (I) steht X für N.

In einer weiteren hervorgehobenen Gruppe von Verbindungen der Formel (I) steht X für CH.

In einer weiteren hervorgehobenen Gruppe von Verbindungen der Formel (I) steht Y für CN.

In einer weiteren hervorgehobenen Gruppe von Verbindungen der Formel (I) steht Y für NO₂.

In einer weiteren hervorgehobenen Gruppe von Verbindungen der Formel (I) steht A für

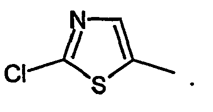

In einer weiteren hervorgehobenen Gruppe von Verbindungen der Formel (I) steht A für

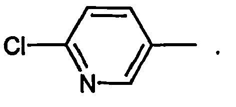

In einer weiteren hervorgehobenen Gruppe von Verbindungen der Formel (I) steht A für

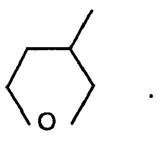

Die oben aufgeführten allgemeinen oder in Vorzugsbereichen aufgeführten Restedefinitionen bzw. Erläuterungen gelten für die Endprodukte und für die Ausgangs-und Zwischenprodukte entsprechend. Diese Restedefinitionen können untereinander, also auch zwischen den jeweiligen Vorzugsbereichen, beliebig kombiniert werden.

Erfindungsgemäß bevorzugt werden die Verbindungen der Formel (I), in welchen eine Kombination der vorstehend als bevorzugt aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

In den oben und nachstehend aufgeführten Restedefinitionen sind Kohlenwasserstoffreste, wie Alkyl - auch in Verbindung mit Heteroatomen wie in Alkoxy - soweit möglich jeweils geradkettig oder verzweigt.

Verwendet man beispielsweise 4-(N-(6-Chlor-3-pyridylmethyl)-N-methyl)amino-2-imino-2,5-dihydro-[1,3]-thiazolin und Bromcyan als Ausgangsstoffe, so kann der Verlauf des erfindungsgemäßen Verfahrens durch das folgende Reaktionsschema wiedergegeben werden:

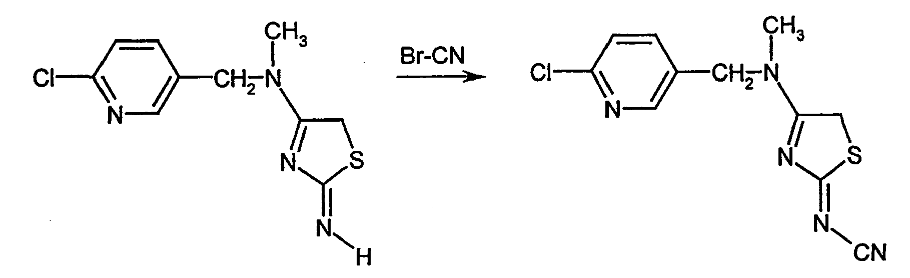

Die Verbindungen der Formel (II) sind neu und auch Gegenstand der vorliegenden Erfindung.

In Abhängigkeit insbesondere von der Bedeutung der Variablen X und Z kommen für die Herstellung der Verbindungen der Formel (II) verschiedene Methoden in Betracht. Auch einige Verbindungen der Formel (I) lassen sich nach weiteren Methoden herstellen.

Zur Erläuterung soll zunächst das folgende Formelschema 1 dienen.

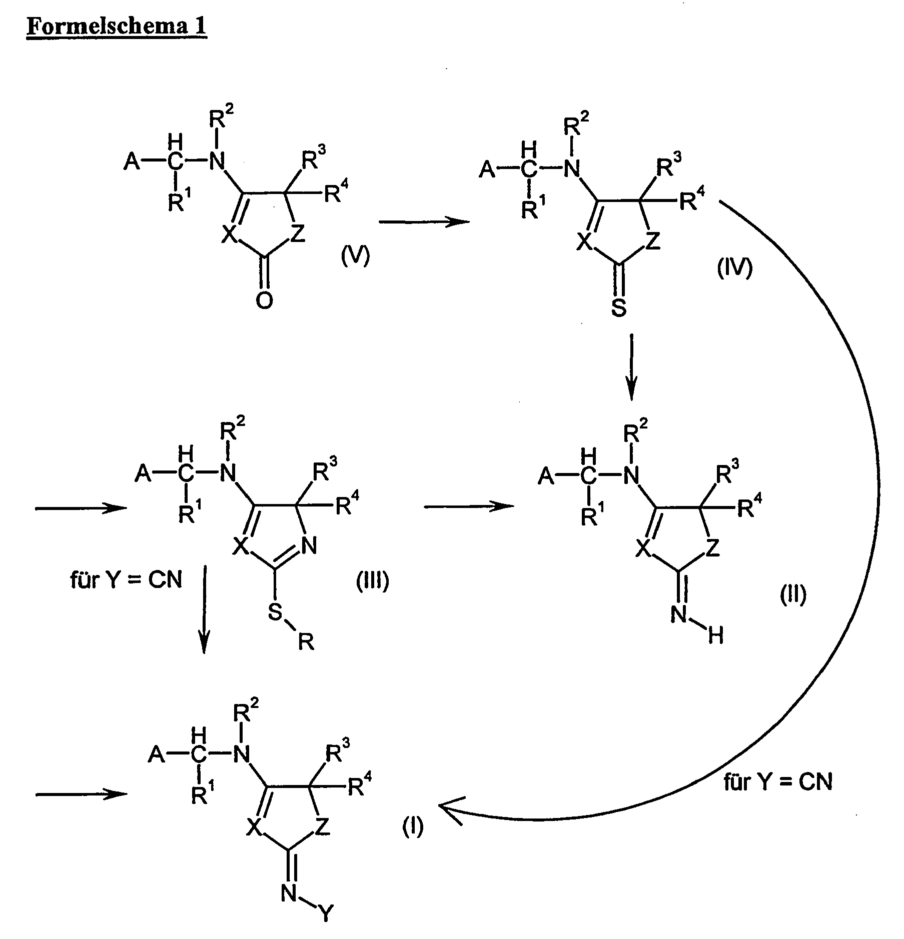

Die im Formelschema 1 beschriebenen Reaktionen lassen sich in allgemein bekannter Weise durchführen.

Die Verbindungen der Formel (V) lassen sich durch Umsetzung mit Schwefelungsreagenzien in die Verbindungen der Formel (IV) überführen.

Eine Vielzahl unterschiedlicher Schweflungsreagenzien ist in der Literatur beschrieben, wie beispielsweise Schwefelwasserstoff (H₂S), Schwefelwasserstoff/Chlorwasserstoff (H₂S/HCl), Wasserstoffpersulfid/Chlorwasserstoff (H₂S₂/HCl), Di-(diethylaluminium)-sulfid [(Et₂Al)₂S], polymeres Ethylaluminiumsulfid [(EtAlS)ₙ], Siliziumdisulfid (SiS₂), Dibortrisulfid (B₂S₃), Phosphorpentachlorid/Dialuminiumtrisulfid/Natriumsulfat (PCl₅/Al₂S₃/Na₂SO₄), Natriumsulfid/Schwefelsäure (Na₂S/H₂SO₄), Diphosphorpentasulfid (P₂S₅), Diphosphorpentasulfid/Pyridin (P₂S₅/Py), Diethylthiocarbamoylchlorid, Diphosphorpentasulfid/Triethylamin (P₂S₅/NEt₃), Diphosphorpentasulfid/n-Butyllithium (P₂S₅/n-BuLi), Diphosphorpentasulfid/Natriumhydrogencarbonat (P₂S₅/NaHCO₃; "Scheeren's Reagens", Bildung von Na²⁺ [P₄S₁₀O]²⁻), Diphosphorpentasulfid/Methanol (P₂S₅/MeOH), SCN-CO-OEt, PSClₓ (NMe₂)₃₋ₓ (X = 0-3), Bis(tricyclohexylzinn)sulfidlBortrihalogenid [(C₆H₁₁)₃Sn]S₂+BX₃ (X=Cl, F), EP 0280867 (1988), Bis(1,5-cyclo-oktandiylboryl)sulfid [(9-BBN)₂S] als Schwefelungsreagens oder als Phosphorpentasulfid-Ersatz 2,4-Bis-(methylthio)-1,3,2,4-dithiadiphosphetan-2,4-disulfid "Davy-Reagens Methyl" (DR-Me), 2,4-Bis-(ethylthio)-1,3,2,4-dithiadiphosphetan-2,4-disulfid "Davy-Reagens Ethyl" (DR-Et), 2,4-Bis-(p-tolylthio)-1,3,2,4-dithiadiphosphetan-2,4-disulfid "Davy-Reagens p-Tolyl oder Heimgartner Reagens" (DR-T), 2,4-Bis-(4-phenoxyphenyl)-2,4-dithioxo-1,3,2,4-dithiadiphosphetan "Belleau's Reagens (BR)", 2,4-Bis-(4-phenylthiophenyl)-2,4-dithioxo-1,3,2,4-dithiadiphosphetan, 2,4-Bis-(4-methoxyphenyl)-2,4-dithioxo-1,3,2,4-dithiadiphosphetan "Lawesson's Reagens (LR)" (vgl. Davy-Reagens: H. Heimgartner et al., Helv. Chim. Acta 70, 1987, S. 1001; Belleau's Reagens: Tetrahedron 40, 1984, S. 2047; Tetrahedron 40, 1984, S. 2663; Tetrahedron Letters 24, 1983, S. 3815; I. Thomson et al., Org. Synth. 62, 1984, S. 158 sowie dort zitierte Literatur; D. Brillon Synthetic Commun. 20 (19), 1990, S. 3085 und dort zitierte Literatur; selektive Thionierung von Oligopeptiden: K. Clausen et al., J. Chem. Soc., Perkin Trans I 1984, 785; O. E. Jensen et al., Tetrahedron 41, 1985, S. 5595; Reviews über "Lawesson's Reagenz, (LR)": R. A. Cherkasov et al., Tetrahedron 41, 1985, S. 2567; M. P. Cava et al., Tetrahedron 41, 1985, S. 5061; Diborylsulfid: Liebigs Ann. Chem. 1992, S. 1081 und dort zitierte Literatur; Metzner et al. in Sulfur Reagents in Organic Synthesis, B. Harcourt: London 1994, Academic Press, S. 44-45).

Alternativ sind auch Reaktionsfolgen, wie beispielsweise eine O-Alkylierung mit R₃O⁺BF₄⁻ (R: -Methyl, Ethyl) (H. Meerwein et al., Justus Liebigs Ann. Chem. 641, (1961) S. 1) und anschließende Umsetzung der Intermediate mit wasserfreiem NaSH (R. E. Eibeck, Anorg. Syn. 7, (1963) S. 128), die *in-situ* Bildung von Chloriminiumsalzen und nachfolgende Reaktion mit Tetrathiomolybdaten, insbesondere Benzyltriethylammoniumtetramolybdat [(Ph-CH₂-NEt₃)₂MoS₄] (Tetrahedron Lett. 36 (45), 1995, S. 8311) oder Hexamethyldisilathian (TMS₂S) (TMS: Trimethylsilyl; P. L. Fuchs et al., J. Org. Chem. 59, 1994, S. 348) möglich.

Zur Durchführung werden als Sulfidierungsreagenzien bevorzugt Phosphorreagenzien wie beispielsweise Diphosphorpentasulfid (P₂S₅), Diphosphorpentasulfid/Pyridin (P₂S₅/Py), Diphosphorpentasulfid/Triethylamin (P₂S₅/NEt₃), Diphosphorpentasulfid/Natriumhydrogencarbonat (P₂S₅/ NaHCO₃ "Scheeren's Reagens") oder besonders bevorzugt das racemisierungsfreie 2,4-Bis-(4-methoxyphenyl)-2,4-dithioxo-1,3,2,4-dithiadiphosphetan (LR: Lawesson's Reagens) (K. Clausen, M. Thorsen, S.-O. Lawesson Tetrahedron 37, 1981, S. 3635) 2,4-Bis-(4-phenoxyphenyl)-2,4-dithioxo-1,3,2,4-dithiadiphosphetan "Belleau's Reagens (BR)" bzw. 2,4-Bis-(4-phenylthiophenyl)-2,4-dithioxo-1,3,2,4-dithiadiphosphetan, eingesetzt.

Im allgemeinen ist es vorteilhaft, dieses Verfahren in Gegenwart von Verdünnungsmitteln durchzuführen. Verdünnungsmittel werden vorteilhaft in einer solchen Menge eingesetzt, dass das Reaktionsgemisch während des ganzen Verfahrens gut rührbar bleibt. Als Verdünnungsmittel zur Durchführung des erfindungsgemäßen Verfahrens kommen alle inerten organischen Lösungsmittel in Frage.

Als Beispiele sind zu nennen: Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol; Alkohole wie Methanol, Ethanol, Isopropanol, Butanol; Ether wie Ethylpropylether, Methyl-tert-butylether, n-Butylether, Anisol, Phenetol, Cyclohexylmethylether, Dimethylether, Diethylether, Dipropylether, Diisopropylether, Di-n-butylether, Diisobutylether, Diisoamylether, Ethylenglycoldimethylether, Tetrahydrofuran, Dioxan, Dichlordiethylether und Polyether des Ethy-lenoxids und/oder Propylenoxids: Amine wie Trimethyl-, Triethyl-, Tripropyl-, Tributylamin, N-Methyl-morpholin, Pyridin und Tetramethylendiamin, Nitrokohlenwasserstoffe wie Niromethan, Nitroethan, Nitropropan, Nitrobenzol, Chlornitrobenzol, o-Nitrotoluol; Nitrile wie Acetonitril, Propionitril, Butyronitril, Isobutyronitril, Benzonitril, m-Chlorbenzonitril sowie Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwasserstoffe wie Pentan, Hexan, Heptan, Oktan, Nonan und technische Kohlenwasserstoffe, beispielsweise sogenannte White Spirits mit Komponenten mit Siedepunkten im Bereich beispielsweise von 40°C bis 250°C, Cymol, Benzinfraktionen innerhalb eines Siedepunktintervalles von 70°C bis 190°C, Cyclohexan, Methylcyclohexan, Petrolether, Ligroin, Octan, Benzol, Toluol, Chlorbenzol, Brombenzol, Nitrobenzol, Xylol; Ester wie Methyl-, Ethyl-, Butyl-, Isobutylacetat, sowie Dimethyl-, Dibutyl-, Ethylencarbonat; Amide wie Hexamethylenphosphortriamid, Formamid, N-Methyl-formamid, N,N-Dimethylformamid, N,N-Dipropylformamid, N,N-Dibutylformamid, N-Methyl-pyrrolidin, N-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, N-Formyl-piperidin, N,N'-1,4-Diformylpiperazin; Ketone wie Aceton, Acetophenon, Methylethylketon, Methylbutylketon.

Selbstverständlich kann man das erfindungsgemäße Verfahren auch in Gemischen der genannten Lösungs- und Verdünnungsmittel durchführen.

Die zu verwendenden Verdünnungsmittel sind vom jeweils eingesetzten Schwefelungsreagens abhängig.

Bevorzugte Verdünnungsmittel zur Thionierung sind jedoch aromatische Kohlenwasserstoffe wie Benzol, Toluol, Chlorbenzol, Brombenzol, Nitrobenzol oder Xylol, Ether wie Ethylpropylether, Methyl-tert-butylether, Anisol, Phenetol, Cyclohexylmethylether, Tetrahydrofuran oder Dioxan.

Die Verbindungen der Formel (IV) lassen sich durch Umsetzung mit HgCl₂/(C₂H₅)₃N/H₂NCN direkt in die Verbindungen der Formel (I) überführen, in denen Y für CN steht (Vgl. Can. J. Chem. 1985, 63, 3089 und auch J. Med. Chem. 1988, 31, 264).

Die Verbindungen der Formel (IV) lassen sich ferner durch Aminolyse mit Ammoniak in Gegenwart von Quecksilbersalzen, durch Erhitzen in einem Reaktionsgefäß mit wässrigem Ammoniak in Gegenwart von geeigneten Oxidationsmitteln, wie beispielsweise tert.-Butylhydroperoxid (TBHP), in Verbindungen der Formel (II) überführen (vgl. M.G. Bock et al., J. Med. Chem. 1988, 31, 264-268; N.W. Jacobsen et al., Aust. J. Chem. 1987, 40, 491-499; T. Lindel et al., Tetrahedron Lett. 1997. 38, (52), 8935-8938).

Die Umsetzung der Verbindungen der Formel (IV) (Z = NH) mit Alkyliodiden in Gegenwart von Basen führt zu Verbindungen der Formel (III) (Z = N) (siehe dazu T. Lindel et al., Tetrahedron Lett. 1997. 38, (52), 8935-8938; M.G. Bock et al., J. Med. Chem. 1988, 31, 264-268). In den Verbindungen der Formel (III) steht R beispielsweise für Alkyl, bevorzugt für Methyl. Die Verbindungen der Formel (III) wiederum lassen sich in grundsätzlich bekannter Weise durch Umsetzung mit einem Gemisch aus Ammoniak/Ammoniumchlorid in Verbindungen der Formel (II) überführen (vgl. T. Lindel et al., Tetrahedron Lett. 1997. 38, (52), 8935-8938).

Die Verbindungen der Formel (II) lassen sich mit Cyanierungsreagenzien bzw. Nitrierungsreagenzien in Verbindungen der Formel (I) überführen.

Als Cyanierungsreagenz kommt beispielsweise Bromcyan (BrCN) in Frage. Die Umsetzung wird in allgemein bekannter Weise durchgeführt (vgl. auch US 4 098 791 und DE 29 16 140).

Nitrierungen sind nach üblichen Verfahren, wie sie beispielsweise in Houben-Weyl, Methoden der Organischen Chemie, Band XI/2 (Georg Thieme Verlag - Stuttgart 1958), S. 99-116, beschrieben sind, durchführbar. Als Nitrierungsreagenzien seien rauchende oder 100 %ige Salpetersäure (Darstellung wasserfreier Salpetersäure (vgl. F.D. Chattaway, Soc. 97, 2100 (1910), gegebenenfalls in Gegenwart von Schwefelsäure (W. J. Middleton et al., J. Heterocycl. Chem. 7, 1045-1049 (1970); L. W. Deady et al., Aust. J. Chem. 35 (10), 2025-2034 (1982); EP 0192060) oder die Verwendung von Salpetersäureestem, Acetylnitrat oder Nitroniumtetrafluoroborat genannt. Bevorzugt wird die Umsetzung mit Acylnitrat in allgemein bekannter Weise durchgeführt.

### Verbindungen der Formel (Va)

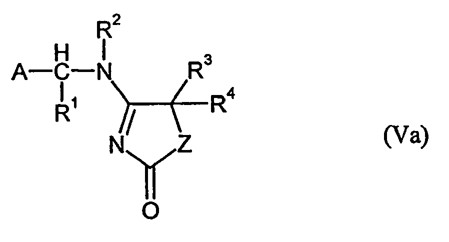

in welcher
- A, R¹, R², R³ und R⁴: die oben angegebenen Bedeutungen haben und
- Z: für O oder CH₂ steht
erhält man beispielsweise, indem man Amine der Formel (VI) 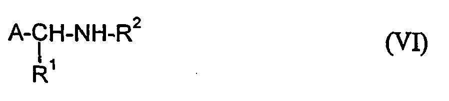 in welcher
- A, R¹ und R²: die oben angegebenen Bedeutungen haben
mit Verbindungen der Formel (VII) 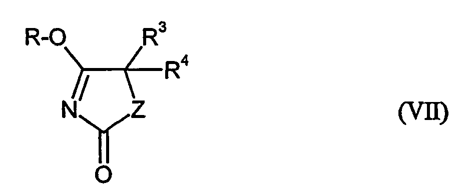 in welcher
- R³, R⁴ und Z: die oben angegbenen Bedeutungen haben und
- R: für Wasserstoff, Methyl oder Ethyl steht,
in Gegenwart eines Verdünnungsmittels wie z.B. Methanol oder Ethanol bei Temperaturen zwischen 0°C und 78°C umsetzt.

Verbindungen der Formel (VII) und Verfahren zu ihrer Herstellung sind beispielsweise bekannt aus Org. Synth. (1980), 59, 132 (für Z = CH₂).

### Man erhält Verbindungen der Formel (Vb)

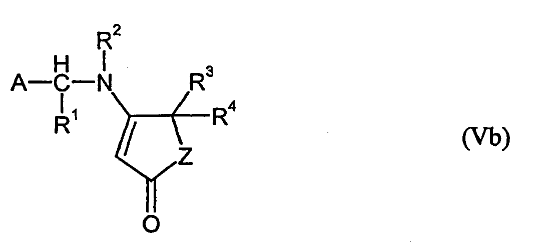

in welcher
- A, R¹, R², R³ und R⁴: die oben angegebenen Bedeutungen haben und
- Z: für CH₂, S oder NR⁵ steht und
- R⁵: die oben angegebene Bedeutung hat,
beispielsweise, indem man Amine der Formel (VI) 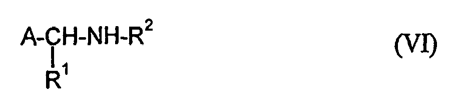 in welcher
- A, R¹ und R²: die oben angegebenen Bedeutungen haben
mit Verbindungen der Formel (VIII) 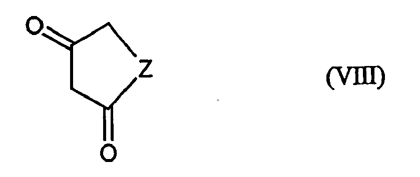 in welcher
- Z: für CH₂, S oder NR⁵ steht und
- R⁵: die oben angegebene Bedeutung hat
in Gegenwart eines Verdünnungsmittels wie beispielsweise Acetonitril, Ethanol, Benzol, Toluol, Chlorbenzol, Brombenzol, Nitrobenzol oder Xylol und gegebenenfalls in Gegenwart eines sauren Katalysators, wie beispielsweise para-Toluolsulfonsäure umsetzt.

Bevorzugte Verdünnungsmittel sind aromatische Kohlenwasserstoffe wie Benzol, Toluol oder Xylol.

Die Umsetzung erfolgt bei Temperaturen zwischen -10°C und +200°C, bevorzugt zwischen 0°C und +150°C, besonders bevorzugt bei Temperaturen zwischen +10°C und +130°C oder bei Siedetemperatur eines geeigneten Verdünnungsmittels. Es kann grundsätzlich unter Normaldruck gearbeitet werden. Vorzugsweise arbeitet man unter Normaldruck und unter Entfernung von Wasser, beispielsweise mittels Wasserabscheider oder in Gegenwart von Molsieb.

Die Verbindungen der Formel (VIII) können auch in der Enol-Form vorliegen. Zu den Verbindungen der Formel (VIII) vgl. z.B. Arch. Pharm. (Weinheim, Ger.), 321(7), 439, 1988; Aust. J. Chem. 38(12), 1847, 1985; Bull. Soc. Chim. Fr., 133(6), 625, 1996; Synthesis (2), 176, 1991; Tetrahedron Lett., 30(29), 3865, 1989; Can. J. Chem., 67(2), 213, 1989.

Man erhält Verbindungen der Formel (IIIa) 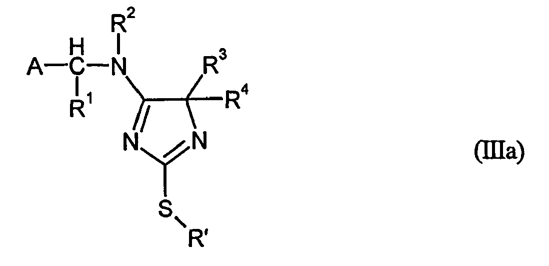 in welcher
- A, R¹, R², R³ und R⁴: die oben angegebenen Bedeutungen haben und
- R': für Alkyl, bevorzugt Methyl steht
beispielsweise, indem man Verbindungen der Formel (IX) 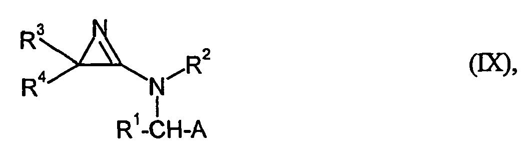 in welcher
- A, R¹, R², R³ und R⁴: die oben angegebenen Bedeutungen haben,
zunächst in einem Verdünnungsmittel wie z.B. einem Ether mit Trimethylsilylisothiocyanat umsetzt und das gebildete Zwischenprodukt anschließend beispielsweise mit Methanol solvolysiert und die entstandene Zwischenstufe der Formel (IIa) 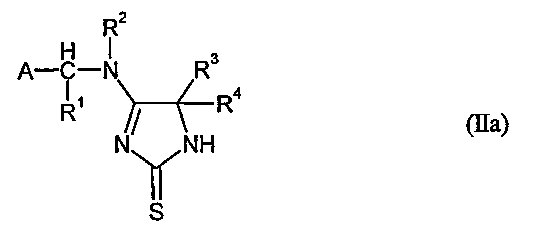 in welcher
- A, R¹, R², R³ und R⁴: die oben angegebene Bedeutung haben,
anschließend zunächst mit einem Alkylhalogenid, bevorzugt einem Alkyliodid, insbesondere Methyliodid, in Gegenwart eines Verdünnungsmittels wie Aceton umsetzt und dann mit einer Base wie Natriumcarbonat in Gegenwart eines Verdünnungsmittels wie Aceton das Endprodukt der Formel (IIIa) freisetzt.

Die Verbindungen der Formel (IX) sind neu und auch Gegenstand der vorliegenden Erfindung.

Man erhält die Verbindungen der Formel (IX) beispielsweise nach folgenden Schema (vgl. Herstellungsbeispiele)

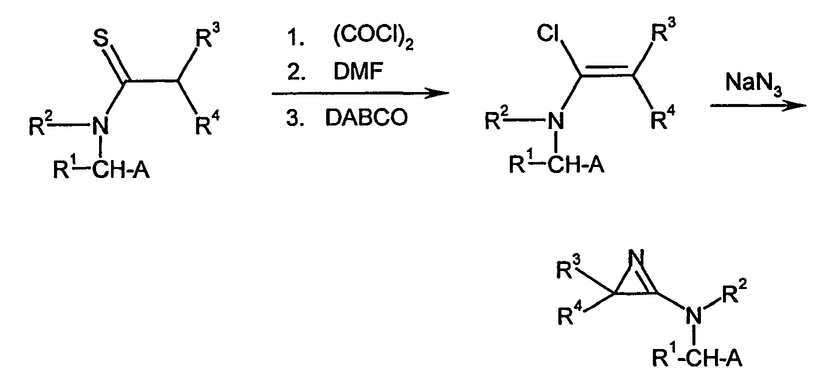

Man erhält ferner Verbindungen der Formel (IIb) 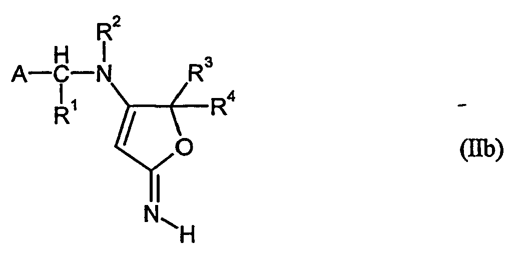 in welcher
- A, R¹, R², R³ und R⁴: die oben angegebenen Bedeutungen haben,
indem man Verbindungen der Formel (VI) 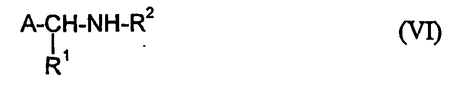 in welcher
- A, R¹ und R²: die oben angegebenen Bedeutungen haben
mit Verbindungen der Formel (XII) 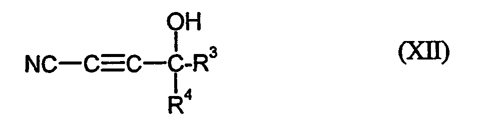 in welcher
- R³ und R⁴: die oben angegebenen Bedeutungen haben,
in Gegenwart eines Verdünnungsmittels wie Methylenchlorid, Methanol oder Acetonitril umsetzt (vgl. J. Chem. Soc., Perkin Trans. 1(5), 1201, 1991; Zh. Org. Khim., 27(3), 526, 1991).

Die Verbindungen der Formel (III) lassen sich auch direkt in solche Verbindungen der Formel (I) überführen, in welchen Y für CN steht. Geeignete Methoden sind z.B. angegeben in JP 7126483 und Arch. Pharm., 303 (8), 625-633 (1970), deren Inhalt ausdrücklich Bestandteil dieser Anmeldung sein soll.

Weiterhin erhält man Verbindungen der Formel (Ia) 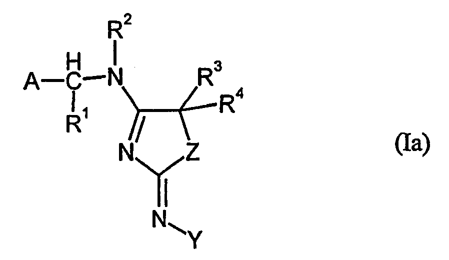 in welcher
- A, R¹, R², R³, R⁴ und Y: die oben angegebenen Bedeutungen haben und
- Z: für S steht,
beispielsweise, indem man Verbindungen der Formel (X) 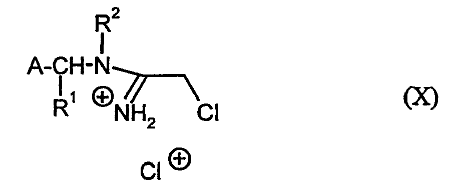 in welcher
- A, R¹ und R²: die oben angegebenen Bedeutungen haben,
mit Kalium-N-cyan(nitro)immomethylsulfanylmethantmolat der Formel (XI) 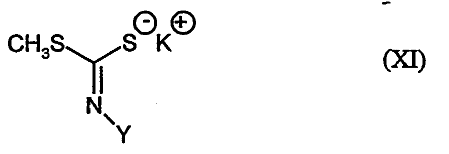 in welcher
- Y: die oben angegebene Bedeutung hat
in Gegenwart eines Verdünnungsmittels umsetzt.

Verbindungen der Formel (I), in welchen Z für S steht lassen sich auch nach J. Heterocyclic Chem. 25, 1849-1856 (1988) gemäß folgender Reaktionsgleichung herstellen:

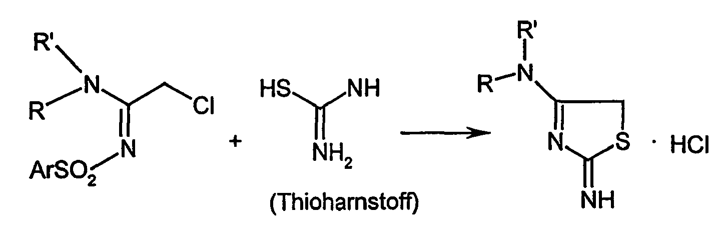

Man erhält die Verbindungen der Formel (X) beispielsweise, indem man Verbindungen der Formel (VI) 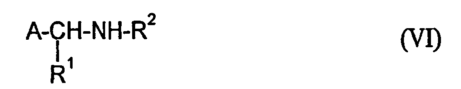 in welcher
- A, R¹ und R²: die oben angegebenen Bedeutungen haben,
in Gegenwart eines Verdünnungsmittels wie Methanol und in Gegenwart einer Base wie Natriummethanolat mit Chloracetonitril umsetzt.

Die Verbindungen der Formel (I), in welchen Z für SO oder SO₂ steht, lassen sich aus den Verbindungen der Formel (I), in welchen Z für S steht, durch Oxidation nach üblichen Verfahren, beispielsweise mit geeigneten Oxidationsmitteln, wie Peroxiden z.B. Wasserstoffperoxid, tert.-Butylperoxid, organische und anorganische Peroxide oder deren Salze, wie 3-Chlorperbenzoesäure, Peressigsäure, Perameisensäure, Dibenzoylperoxid, Permanganat, oder mit einem Gemisch aus Kaliumperoxomonosulfat, 2 KHSO₅, KHSO₄ und einem Lösungsmittel oder Lösungsmittelgemisch (z.B. Wasser, Essigsäure, Methanol, Methylenchlorid) erhalten.

Man kann das Peroxid auch in-situ aus einem anderen Peroxid, beispielsweise Peressigsäure an Essigsäure und Wasserstoffperoxid herstellen (vgl. auch A.R. Katritzky, C.W. Rees in Comprehensive Heterocyclic Chemistry, Pergamon Press, Oxford, New York, 1984, Vol. 3, S. 96; D.J. Brown et al. Chem. Soc. (c), 1971, S. 256).

Die Oxidation kann auch mittels geeigneter Katalysatoren gestartet oder beschleunigt werden.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spp..
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Acheta domesticus, Gryllotalpa spp., Locusta migratoria migratorioides, Melanoplus spp., Schistocerca gregaria.
Aus der Ordnung der Blattaria z.B. Blatta orientalis, Periplaneta americana, Leucophaea maderae, Blattella germanica.
Aus der Ordnung der Dermaptera z.B. Forficula auricularia.
Aus der Ordnung der Isoptera z.B. Reticulitermes spp..
Aus der Ordnung der Phthiraptera z.B. Pediculus humanus corporis, Haematopinus spp., Linognathus spp., Trichodectes spp., Damalinia spp..
Aus der Ordnung der Thysanoptera z.B. Hercinothrips femoralis, Thrips tabaci, Thrips palmi, Frankliniella accidentalis.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp..
Aus der Ordnung der Siphonaptera z.B. Xenopsylla cheopis, Ceratophyllus spp..
Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp..
Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp..

Die erfindungsgemäßen Verbindungen der Formel (I) zeichnen sich insbesondere durch Wirkung gegen saugende Insekten aus.

Die erfindungsgemäßen Verbindungen können gegebenenfalls in bestimmten Konzentrationen bzw. Aufwandmengen auch als Herbizide und Mikrobizide, beispielsweise als Fungizide, Antimykotika und Bakterizide verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Emtegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran,
Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil,
Pyroquilon, Pyroxyfur,
Quinconazol, Quintozen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol,
Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-b-propyl-1H-1,2,4-triazol-1-ethanol,
α-(2,4-Dichlorphenyl)-β-methoxy-a-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-a-(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester
1-(2,4-Dichlorphenyl)-2-(1H-1,2,4-triazol-1-yl)-ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
2-[(1-Methylethyl)-sulfonyl]-5-(trichlormethyl)-1,3,4-thiadiazol,
2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)-oxy]-methyl]-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)-oxy]-2,5-thiophendicarboxylat,
cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholin-hydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Formyl-N-hydroxy-DL-alanin -Natriumsalz,
O,O-Diethyl-[2-(dipropylamino)-2-oxoethyl]-ethylphosphoramidothioat,
O-Methyl-S-phenyl-phenylpropylphosphoramidothioate,
S-Methyl-1,2,3-benzothiadiazol-7-carbothioat,
spiro[2H]-1-Benzopyran-2,1'(3'H)-isobenzofuran]-3'-on,

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin, Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, BPMC, Bromophos A, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Cis-Resmethrin, Cispermethrin, Clocythrin, Cloethocarb, Clofentezine, Cyanophos, Cycloprene, Cycloprothrin, Cyfluthrin, Cyhalothrin, Cyhexatin, Cypermethrin, Cyromazine,
Deltamethrin, Demeton M, Demeton S, Demeton-S-methyl, Diafenthiuron, Diazinon, Dichlorvos, Diflubenzuron, Dimethoat, Dimethylvinphos, Diofenolan, Disulfoton, Docusat-sodium, Dofenapyn,
Eflusilanate, Emamectin, Empenthrin, Endosulfan, Entomopfthora spp., Eprinomectin, Esfenvalerate, Ethiofencarb, Ethion, Ethoprophos, Etofenprox, Etoxazole, Etrimfos,
Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazinam, Fluazuron, Flubrocythrinate, Flucycloxuron, Flucythrinate, Flufenoxuron, Flutenzine, Fluvalinate, Fonophos, Fosmethilan, Fosthiazate, Fubfenprox, Furathiocarb,
Granuloseviren
Halofenozide, HCH, Heptenophos, Hexaflumuron, Hexythiazox, Hydroprene,
Imidacloprid, Isazofos, Isofenphos, Isoxathion, Ivermectin,
Kernpolyederviren
Lambda-cyhalothrin, Lufenuron
Malathion, Mecarbam, Metaldehyd, Methamidophos, Metharhizium anisopliae, Metharhizium flavoviride, Methidathion, Methiocarb, Methomyl, Methoxyfenozide, Metolcarb, Metoxadiazone, Mevinphos, Milbemectin, Monocrotophos,
Naled, Nitenpyram, Nithiazine, Novaluron
Omethoat, Oxamyl, Oxydemethon M
Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propoxur, Prothiofos, Prothoat, Pymetrozine, Pyraclofos, Pyresmethrin, Pyrethrum, Pyridaben, Pyridathion, Pyrimidifen, Pyriproxyfen,
Quinalphos,
Ribavirin
Salithion, Sebufos, Selamectin, Silafluofen, Spinosad, Sulfotep, Sulprofos,
Tau-fluvalinate, Tebufenozide, Tebufenpyrad, Tebupirimiphos, Teflubenzuron, Tefluthrin, Temephos, Temivinphos, Terbufos, Tetrachlorvinphos, Thetacypermethrin, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
Vamidothion, Vaniliprole, Verticillium lecanii
YI 5302
Zeta-cypermethrin, Zolaprofos
(1R-cis)-[5-(Phenylmethyl)-3-furanyl]-methyl-3-[(dihydro-2-oxo-3(2H)-furanyliden)-methyl]-2,2-dimethylcyclopropancarboxylat
(3-Phenoxyphenyl)-methyl-2,2,3,3-tetramethylcyclopropanecarboxylat
1-[(2-Chlor-5-thiazolyl)methyl]tetrahydro-3,5-dimethyl-N-nitro-1,3,5-triazin-2(1H)-imin
2-(2-Chlor-6-fluorphenyl)-4-[4-(1,1-dimethylethyl)phenyl]-4,5-dihydro-oxazol 2-(Acetyloxy)-3-dodecyl-1,4-naphthalindion
2-Chlor-N-[[[4-(1-phenylethoxy)-phenyl]-amino]-carbonyl]-benzamid
2-Chlor-N-[[[4-(2,2-dichlor-1,1-difluorethoxy)-phenyl]-amino]-carbonyl]-benzamid 3-Methylphenyl-propylcarbamat
4-[4-(4-Ethoxyphenyl)-4-methylpentyl]-1-fluor-2-phenoxy-benzol
4-Chlor-2-(1,1-dimethylethyl)-5-[[2-(2,6-dimethyl-4-phenoxyphenoxy)ethyl]thio]-3(2H)-pyridazinon
4-Chlor-2-(2-chlor-2-methylpropyl)-5-[(6-iod-3-pyridinyl)methoxy]-3(2H)-pyridazinon
4-Chlor-5-[(6-chlor-3-pyridinyl)methoxy]-2-(3,4-dichlorphenyl)-3(2H)-pyridazinon
Bacillus thuringiensis strain EG-2348
Benzoesäure [2-benzoyl-1-(1,1-dimethylethyl)-hydrazid
Butansäure 2,2-dimethyl-3-(2,4-dichlorphenyl)-2-oxo-1-oxaspiro[4.5]dec-3-en-4-yl-ester
[3-[(6-Chlor-3-pyridinyl)methyl]-2-thiazolidinyliden]-cyanamid
Dihydro-2-(nitromethylen)-2H-1,3-thiazine-3(4H)-carboxaldehyd
Ethyl-[2-[[1,6-dihydro-6-oxo-1-(phenylmethyl)-4-pyridninyl]oxy]ethyl]-carbamat N-(3,4,4-Trifluor-1-oxo-3-butenyl)-glycin
N-(4-Chlorphenyl)-3-[4-(difluormethoxy)phenyl]-4,5-dihydro-4-phenyl-1H-pyrazol-1-carboxamid
N-[(2-Chlor-5-thiazolyl)methyl]-N'-methyl-N"-nitro-guanidin
N-Methyl-N'-(1-methyl-2-propenyl)-1,2-hydrazindicarbothioamid
N-Methyl-N'-2-propenyl-1,2-hydrazindicarbothioamid
O,O-Diethyl-[2-(dipropylamino)-2-oxoethyl]-ethylphosphoramidothioat

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Stemostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine hervorragende Wirksamkeit gegen Aphis spp. und Myzus spp.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpudems sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:

### Käfer wie

Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.

### Hautflügler wie

Sirex juvencus, Urocerus gigas, Urocerus gigas taignus, Urocerus augur.

### Termiten wie

Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

### Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:

Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindelöl und/oder Monochlomaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der- vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällen vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethem wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyphenoxid und Triflumuron,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflusskrebse) zusammengefasst werden, besondere Bedeutung zu.

Es wurde nun überraschenderweise gefunden, dass die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis-(trialkylzinn)-sulfiden, Tri-*n*-butylzinnlaurat, Tri-*n*-butylzinnchlorid, Kupfer(I)-oxid, Triethylzinnchlorid, Tri-*n*-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-*n*-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zink oxid, Kupfer(I)-ethylen-bisdithiocarbamat, Kupferthiocyanat, Kupfemaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Andfouling-Mittel eignen sich vorzugsweise:

### Algizide wie

2-*tert.*-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;

### Fungizide wie

Benzo[*b*]thiophencarbonsäurecyclohexylamid-S,S-dioxid, Dichlofluanid, Fluorfolpet, 3-Iod-2-propinyl-butylcarbamat, Tolylfluanid und Azole wie Azaconazole, Cyproconazole, Epoxyconazole, Hexaconazole, Metconazole, Propiconazole und Tebuconazole;

### Molluskizide wie

Fentinacetat, Metaldehyd, Methiocarb, Niclosamid, Thiodicarb und Trimethacarb; oder herkömmliche Antifouling-Wirkstoffe wie 4,5-Dichlor-2-octyl-4-isothiazolin-3-on, Diiodmethylparatrylsulfon, 2-(N,N-Di-methylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten des weiteren die üblichen Bestandteile wie z.B. in Ungerer, *Chem. Ind.* **1985,** 37, 730-732 und Williams, Antifouling Marine Coatings, Noyes, Park Ridge, **1973** beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wässrigen System, Vinylchlorid/Vinylacetat-Copolymersysteme in Form wässriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfem, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Herstellungsbeispiele

### Beispiel I-1

### 4-(N-(6-Chlor-3-pyridylmethyl)-N-methyl)amino-2-cyanimino-2,5-dihydro-[1,3]-thiazolin

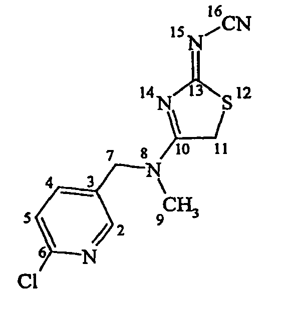

0.75 g (10.0 mmol) Chloracetonitril werden in eine auf 5°C gekühlte Lösung von 0.03 g (1.3 mmol) Natrium in 20 ml abs. Methanol getropft. Nach beendeter Zugabe wird eine Stunde bei dieser Temperatur unter Feuchtigkeitsausschluss gerührt. Zu dieser Lösung werden 1.93 g (10.0 mmol) N,N-methyl-(6-Chlor-3-pyridylmethyl)-amin-hydrochlorid in 60 ml abs. Methanol innerhalb von einer Stunde getropft, das Reaktionsgemisch auf Raumtemperatur gebracht und 20 Stunden gerührt. Unter vermindertem Druck wird das Lösungsmittel bei Raumtemperatur am Rotationsverdampfer vollständig entfernt. Der ölige Rückstand wird ohne weitere Aufarbeitung in 40 ml Aceton gelöst, mit 1.70 g (10.0 mmol) Kalium-*N*-cyaniminomethylsulfanylmethanthiolat versetzt und fünf Stunden bei Raumtemperatur gerührt. Das Reaktionsgemisch wird am Rotationsverdampfer eingeengt, wobei das Produkt auskristallisiert. Der Rückstand wird filtriert und aus Ethanol umkristallisiert.

| | | |
|---|---|---|
| Ausbeute | : 0.42 g (15 % d. Th.) | C₁₁H₁₀CN₅S (M = 279.75 g / mol) |

MS (FAB / NBA-Matrix)
m / z (%) = 283 (5), 282 (31), 281 (15), 280 (80) [M + 1]⁺, 279 (7).
IR (fest in KBr)
ν̃/ cm⁻¹ = 2971 (C-H _{alkyl}), 2180 (C≡N), 1625 (C=N), 1588 u. 1566 u. 1458 (Ringschw.), 1500 (C=N), 1407, 1381, 1300, 1264, 1221, 1097 (C_{aryl}-Cl), 879, 835.

### Beispiel I-2

### 4-(N-(2-Chlor-thiazol-5-yl-)-Nethyl)amino-2-cyanimino-2,5-dihydro-[1,3]-thiazolin.

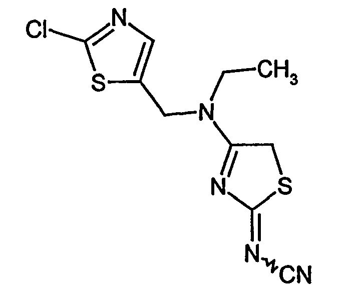

Die Ringschlussreaktion erfolgt analog der Reaktionsvorschrift des Beispiels I-1 unter Verwendung von:

| | |
|---|---|
| 5,30 g (30 mMol) | N-Ethyl-N-(2-chlor-thiazol-5-yl-methyl)amin *(vgl. EP 302389 A2, 1989)* |
| 2,26 g (30 mMol) | Chloracetonitril |
| 5,11 g (30 mMol) | Kalium-N-cyaniminomethylsulfanylmethanthiolat |
| 0,10 g | Natrium |
| 240,9 ml | Methanol |
| 120,5 ml | Aceton |

Das zurückbleibende Rohprodukt wird mit dem Fließmittel Wasser (A) / Methanol (B) (Programm - Start: 50:50 / Schritt 1: 90 % A, 10 min / Schritt 2: 90 % bis 10 % A, 90 min / Schritt 3 : 10 % A, 10 min; Pumpenhub ca. 40 %) mittels Reverse-Phase (RP)-Chromatographie vorfraktioniert. Eine zweite Auftrennung des zuvor erhaltenen Produktes (1,3 g) und die sich anschließende präparative HPLC führt zum reinen 4-(*N*-(2-Chlor-thiazol-5-yl-)-N-ethyl)amino-2-eyanimino-2,5-dihydro-[1,3]-thiazolin als E/Z-Isomerengemisch (vgl. NMR).
C₁₀H₁₀ClN₅S₂ (M = 299,8 g/mol)
LC-MS (ESI-Positiv) (m/z, %) = 300 (MH⁺, 100)
Rₜ= 3,54 min (0,1 % HCOOH / Acetonitril)
¹H-NMR (600 MHz, CD₃CN, TMS) δ = 1,25 (3H, -CH₃); 3,50 (2H, -CH₂-); 4,48
(2H, -S-CH₂-); 4,90 (2H, -N-CH₂-Ct); 7,61 (1H, Ct-H) ppm.
¹³C- mit ¹H-Entkopplung und ¹H-/¹³C-Korrelation (HMBC, HMQC, CD₃CN, TMS) δ = 13,0 (-CH₃); 40,1 (-S-CH₂-); 40,8 (-S-CH₂-); 46,5 (-CH₂-); 46,6 (-CH₂-); 47,5 (-N-CH₂-); 117,9 (-CN); 135,6 (Ct-C); 142,5 (Ct-C); 153,5 (Ct-C-Cl); 178,7 (-N-C=N-); 181,4 (-N-C=N-); 191,0 (-C=N-); 191,9 (-C=N-) ppm. (E/Z-Isomerengemisch)
HPLC (Wasser / Acetonitril) = 3,17 min; λₘₐₓ = 287,5 nm

### Beispiel I-3

### 5-Cyanimino-2-(N-(2-Chlor-thiazol-5-yl-)-N-ethyl)amino-pyrrolin

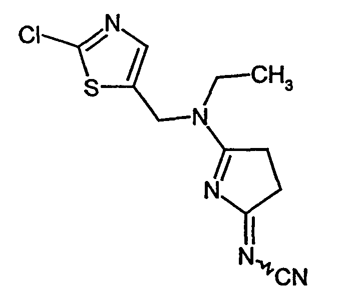

Man verrührt 0,011 g (0,039 mMol) 2-(*N*-(2-Chlor-thiazol-5-yl-)-*N*-ethyl)amino-pyrrolin-5-thion (IV-1), 0,002 g (0,039 mMol) Cyanamid und 0,01 ml (0,039 mMol) Triethylamin in 5,0 ml Methanol und versetzt das Reaktionsgemisch unter Schutzgasatmosphäre (Argon) mit 0,011 g (0,039 mMol) Quecksilber(II)-chlorid. Nach 15 Minuten wird die entstandene Suspension über Kieselgel 60 - Merck (Korngröße: 0,04 bis 0,063 mm) filtriert und das Filtrat im Vakuum eingeengt. Der verbleibende Rückstand wird mit dem Fließmittel Wasser (A) / Methanol (B) (Programm - Start: 50:50 / Schritt 1: 90 % A, 10 min / Schritt 2: 90 % bis 10 % A, 90 min / Schritt 3: 10 % A, 10 min; Pumpenhub ca. 40 %) mittels Reverse-Phase (RP)-Chromatographie aufgetrennt. Von den vier erhaltenen Fraktionen wurden die Fraktionen zwei bis vier nochmals mit dem Fließmittel Wasser (A) / Methanol (B) (Programm - Start: 50:50 / Schritt 1: 90 % A, 10 min / Schritt 2: 90 % bis 10 % A, 90 min / Schritt 3: 10 % A, 10 min; Pumpenhub ca. 40 %) mittels RP-Chromatographie gereinigt.
Ausbeute: 1,4 mg (0,05 % d. Th.) C₁₁H₁₂ClN₅S (M = 281,7 g/mol)
LC-MS (ESI-Positiv) (m/z, %) = 282 (MH⁺, 100)
Rₜ = 2,93 min (0,1 % HCOOH / Acetonitril)

Analog den Beispielen I-1 bis I-3 können die in der nachstehenden Tabelle I aufgeführten Verbindungen der allgemeinen Formel (I) hergestellt werden.

**Tabelle I**

| Beispiele für Verbindungen der Formel (I) | | | | | | | | | |
|---|---|---|---|---|---|---|---|---|---|
| 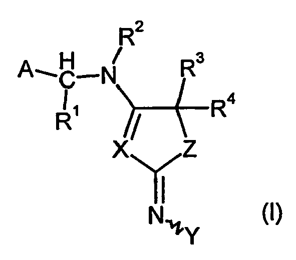 | | | | | | | | | |

| Bsp. Nr. | A | R¹ | R² | R³ | R⁴ | X | Y | Z | Physikalische Daten^{a)} |
|---|---|---|---|---|---|---|---|---|---|
| I-4 | 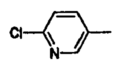 | -H | -Et | -H | -H | -N= | -CN | -S- | 294 (MH⁺, 100); 3,73 min |

| | | | | | | | | | |
|---|---|---|---|---|---|---|---|---|---|
| ^{a)} LC-MS (ESI-Positiv) (m/z, %); Rₜ (in min) (0,1 % HCOOH / Acetonitril) | | | | | | | | | |

### Beispiel III-1

### 5-(N-(6-Chlor-3-pyridylmethyl)-N-methylamino)-4,4-dimethyl-2-methylsulfanyl-4H-imidazol

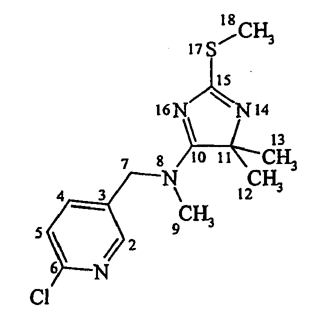

In 80 ml abs. Aceton werden 2.26 g (8.0 mmol) 4-(*N*-(6-Chlor-3-pyridylmethyl)-*N-*methyl)amino)-5,5-dimethyl-2,5-dihydro-1*H*-imidazol-2-thion gelöst, mit 3.40 g (24.0 mmol) Iodmethan in 10 ml abs. Aceton versetzt und vier Stunden bei Raumtemperatur gerührt - bis das Thion abreagiert ist (DC-Kontrolle). Das überschüssige Iodmethan und das Lösungsmittel werden am Rotationsverdampfer unter vermindertem Druck entfernt. Zur Freisetzung der Base wird das verbliebene Öl in 60 ml Aceton aufgenommen und über festem Natriumcarbonat 14 Stunden bei Raumtemperatur gerührt. Zur Isolierung des Produktes wird filtriert, das Lösungsmittel unter vermindertem Druck entfernt und mit Ethylacetat / Methanol 2 / 1 (R_{f} = 0.2) über Kieselgel eluiert. Die freie Base fällt als sehr viskoses Öl an.

| | | |
|---|---|---|
| Ausbeute | : 1.93 g (81 % d. Th.) | C₁₃H₁₇ClN₄S (M = 296.82 g / mol) |

MS (EI / 70 eV)
m / z (%) = 298 (13), 297 (7), 296 (35), 283 (5), 281 (13), 226 (3), 224 (8),170 (24), 128 (11), 126 (33), 115 (24), 100 (100), 99 (10), 89 (16), 82 (15), 74 (14), 58 (30), 56 (11), 43 (96).
¹H - NMR₃₀₀ (DMSO-d₆ / TMS) bei 343 K
δ / ppm = 1.41 (s, 6 H, H-12 u. -13), 2.36 (s, 3 H, H-18), 3.12 (s, 3 H, H-9), 4.73 (s, 2 H, H-7), 7.48 (d, 1 H, H-5, *J =* 8.2 Hz), 7.72 (dd, 1 H, H-4, *J* = 8.2 Hz, *J* = 2.5 Hz), 8.32 (d, 1 H, H-2, *J*= 2.5 Hz).

### Beispiel IV-1

### 2-(N-(2-Chlor-thiazol-5-yl-)-N-ethyl)amino-pyrrolin-5-thion

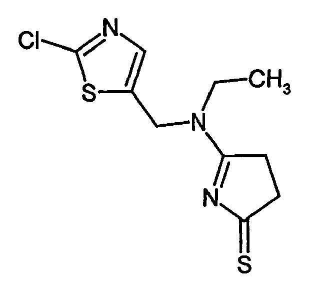

0,5 g (1,94 mMol) 2-(*N*-(2-Chlor-thiazol-5-yl-)-*N*-ethyl)amino-pyrrolin-5-on (V-7) werden in 50 ml Tetrahydrofuran mit 0,81 g (2,00 mMol) 4-Methoxyphenyl-dithiophosphonsäureanhydrid (Lawesson's Reagenz) ca. 18 Stunden bei Raumtemperatur gerührt. Anschließend wird der gesamte Reaktionsansatz über eine Kieselgelsäule (Kieselgel 60 - Merck, Korngröße: 0,04 bis 0,063 mm) filtriert und der Rückstand mit einem Gemisch aus Essigester/ Methanol nachgewaschen. Die organische Phase wird im Vakuum eingeengt und der verbleibende Rückstand mit dem Fließmittel Wasser (A) / Methanol (B) (Programm - Start: 50:50 / Schritt 1: 90 % A, 10 min /Schritt 2: 90 % bis 10 % A, 90 min / Schritt 3: 10 % A, 10 min; Pumpenhub ca. 40 %) mittels Reverse-Phase (RP)-Chromatographie aufgetrennt. Von den vier erhaltenen Fraktionen wurden die Fraktionen zwei bis vier nochmals mit dem Fließmittel Wasser (A) / Methanol (B) (Programm - Start: 50:50 / Schritt 1: 90 % A, 10 min / Schritt 2: 90 % bis 10 % A, 90 min / Schritt 3: 10 % A, 10 min; Pumpenhub ca. 40 %) mittels RP-Chromatographie gereinigt.
Ausbeute: 0,01 g (1,8 % d. Th.) C₁₀H₁₂ClN₃S₂ (M = 273,8 g/mol)
LC-MS (ESI-Positiv) (m/z, %) = 274 (MH⁺, 100)
Rₜ= 3,12 min (0,1 % HCOOH / Acetonitril)
¹H-NMR (400 MHz, CDCl₃, TMS) δ = 1,33 (3H, -CH₃); 2,88 (2H, -CH₂-); 3.23 (2H, -CH₂-); 3.48 (2H, -N-CH₂-); 5,00 (2H, N-CH₂-Ct); 7,52 (1H, Ct-H) ppm.
¹³C- mit ¹H-Entkopplung und ¹H-/¹³C-Korrelation (HMBC, HMQC, CDCl₃, TMS) δ = 13,4 (-CH₃); 30,6 (-CH₂-); 44,9 (-CH₂-); 45,1 (-N-CH₂-); 45,5 (-N-CH₂-Ct); 134,0, (Ct-C); 140,9 (Ct-C); 154,2 (Ct-C-Cl); 185,1 (-N-C=N-); 230,8 (C=S) ppm.

Analog dem Beispiel IV-1 können die in der nachstehenden Tabelle II aufgeführten Verbindungen der allgemeinen Formel (IV) hergestellt werden.

**Tabelle II**

| Beispiele für Verbindungen der Formel (IV) | | | | | | | | |
|---|---|---|---|---|---|---|---|---|
| 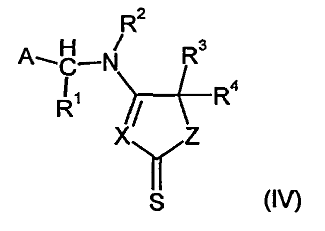 | | | | | | | | |

| Bsp. Nr. | A | R¹ | R² | R³ | R⁴ | X | Z | Physikalische Daten^{a)} |
|---|---|---|---|---|---|---|---|---|
| IV-2 | 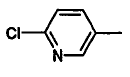 | -H | -Me | -H | -H | -N= | -CH₂- | 254 (MH⁺, 100); 2,49 min |
| IV-3 | 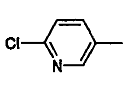 | -H | -Et | -H | -H | -N= | -CH₂- | 268 (MH⁺, 100); 2,73 min |
| IV-4 | 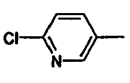 | -H | -Me | -H | -H | -CH= | -S- | 271 (MH⁺, 100); 3,80 min |
| IV-5 | 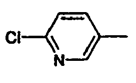 | -H | -Et | -H | -H | -CH= | -S- | 285 (MH⁺, 100); 4,19 min |

| | | | | | | | | |
|---|---|---|---|---|---|---|---|---|
| ^{a)} LC-MS (ESI-Positiv) (m/z, %); Rₜ (in min) (0,1 % HCOOH / Acetonitril) | | | | | | | | |

### Beispiel IV-6

### 4-(N-(6-Chlor-3-pyridylmethyl)-N-methylamino)-5,5-dimethyl-2,5-dihydro-1H-imidazol-2-thion

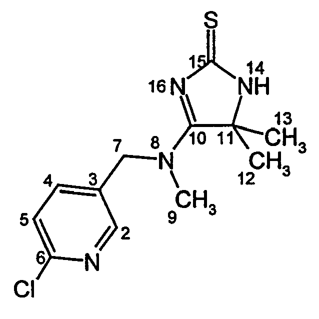

In 30 ml abs. Diethylether werden bei Raumtemperatur 2.24 g (10.0 mmol) 6-Chlor-3-(*N*-(2,2-dimethyl-2*H*-azirin-3-yl)-*N*-methyl)-aminomethylpyridin in einer Stickstoffatmosphäre mit 1.31 g (10.0 mmol) Trimethylsilylisothiocyanat in 10 ml abs. Diethylether tropfenweise versetzt. Nach beendeter Zugabe wird das Reaktionsgemisch 20 Minuten gerührt und zur Hydrolyse mit 4 ml abs. Methanol in 8 ml abs. Diethylether versetzt. Der erhaltene Feststoff wird filtriert, zwei Stunden in Methanol gerührt und wieder filtriert.

| | | |
|---|---|---|
| Ausbeute | : 2.69 g (95 % d. Th.) | C₁₂H₁₅ClN₄S (M = 282.80 g / mol) |

MS (EI / 70 eV)
m / z (%) = 284 (34), 283 (18), 282 (93), 281 (12), 269 (8), 267 (20), 247 (14), 156 (18), 128 (23), 127 (14), 126 (55), 101 (9), 100 (29), 99 (100), 83 (10), 72 (11), 58 (17), 42 (74), 41 (25).
¹H - NMR₃₀₀ (DMSO-d₆ / TMS)
δ / ppm = 1.59 (s, 6 H, H-12 u. H-13), 1.65 (s, 4.8 H, H-12 u. H-13)*, 3.18 (s, 2.4 H, H-9)*, 3.23 (s, 3 H, H-9), 4.85 (s, 2 H, H-7), 5.01 (s, 1.6 H, H-7)*, 7.58 (t, 3.8 H, H-5, *J =* 8.8 Hz), 7.84 (dd, 1 H, H-4, *J* = 7.8 Hz, *J* < 1 Hz), 7.93 (dd, 0.8 H, H-4, *J* = 8.1 Hz, *J* < 1 Hz)*, 8,42 (d, 1 H, H-2, *J* < 1 Hz), 8.47 (d, 0.8 H, H-2, *J <* 1 Hz)*, 10.09 (br, 1 H, H-14), 10.70 (br, 0.8 H, H-14)*.

### Beispiel V-1

### 2-(N-(2-Chlorpyrid-5-yl-)-N-methyl)amino-pyrrolin-5-on

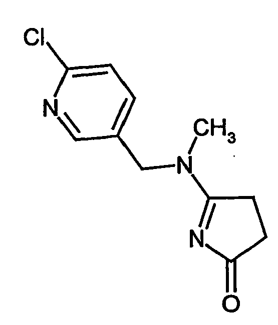

3,70 g (29,0 mMol) 2-Ethoxy-pyrrolin-5-on *(vgl. K. Matoba et al., Chem. Pharm. Bull. 1974, 22(12), 2999-3001; G. Crockett et al., Org. Synth. 1980, 59, 132-40)* werden in 60 ml Methanol gelöst, mit 4,4 g (29,0 mMol) N-Methyl-N-(2-chlor-pyrid-5-yl-methyl)amin (vgl. EP 302389 A2, 1989) versetzt und 5 Tage bei Raumtemperatur gerührt. Anschließend wird das gesamte Reaktionsgemisch im Vakuum eingeengt. Die zurückbleibenden Kristalle werden mit 25 ml Ethanol verrührt und abgetrennt.
Ausbeute: 4,6 g (66,7 % d. Th.) C₁₁H₁₂ClN₃O (M = 237,7 g/mol)
LC-MS (ESI-Positiv) (m/z, %) = 238 (MH⁺, 100)
Rₜ= 1,08 min (0,1 % HCOOH / Acetonitril)
¹H-NMR (600 MHz, CDCl₃, TMS) δ = 2,66 (2H, -CH₂-); 2,84 (2H, -CH₂-); 3,08 (3H, -N-CH₃); 4,88 (2H, -CH₂-Cl-Py); 7,31 (1H, Cl-Py-H); 7,78 (1H, Cl-Py-H); 8,34 (1H, Cl-Py-H) ppm.
¹³C- mit ¹H-Entkopplung und ¹H-/¹³C-Korrelation (600 MHz, HMBC, HMQC, CDCl₃, TMS) δ = 28,4 (-CH₂-); 31,9 (-CH₂-); 35,5 (-N-CH₃); 51,4 (-N-CH₂-Cl-Py); 124,5 (Cl-Py-C), 139,3 (Cl-Py-C); 149,2 (Cl-Py-C); 151,2 (Cl-Py-C-Cl); 185,5 (-C=); 192,7 (C=O) ppm.
HPLC (0.05M NH₄HCO₃ / Acetonitril) = 1,41 min; λₘₐₓ = 238 nm

### Beispiel V-2

### 4-(N-(2-Chlor-thiazol-5-yl-)-N-methyl)amino-2(5H)-thiophenon

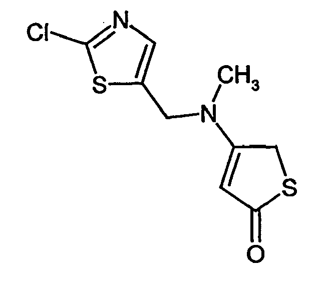

1,32 g (11,4 mMol) 4-Hydroxy-2(5H)-thiophenon *(EP 189096 A1, 1986)* und 1,85 g (11,4 mMol) N-Methyl-N-(2-chlor-thiazol-5-yl-methyl)amin *(vgl. EP 302389 A2, 1989)* werden in 57 ml Toluol in Gegenwart von 17,1 g Molekularsieb 4A unter Schutzgas (Argon) 12 Stunden bei Rückflusstemperatur am Wasserabscheider gerührt. Anschließend wird der gesamte Reaktionsansatz im Vakuum eingeengt und der verbleibende Rückstand über eine Kieselgelsäule (Kieselgel 60 - Merck, Korngröße: 0,04 bis 0,063 mm) mit dem Fließmittel Essigester aufgetrennt.
Ausbeute: 0,20 g (6,4 % d. Th.) C₉H₉ClN₂OS₂ (M = 260,7 g/mol)
LC-MS (ESI-Positiv) (m/z, %) = 261 (MH+, 100)
Rₜ= 3,33 min (0,1 % HCOOH / Acetonitril)
¹H-NMR (400 MHz, CD₃CN, TMS) δ = 2,99 (3H, -CH₃); 4,06 (2H, -CH₂-); 4,61 (2H, -N-CH₂-Ct); 5,15 (1H, -CH=); 7,50 (1H, Ct-H) ppm.
HPLC (0, 1 % H₃PO₄ / Acetonitril) = 2,71 min; λₘₐₓ = 242, 298 nm

Analog den Beispielen V-1 und V-2 können die in der nachstehenden Tabelle III aufgeführten Verbindungen der allgemeinen Formel (V) hergestellt werden.

**Tabelle III**

| Beispiele für Verbindungen der Formel (V) | | | | | | | | |
|---|---|---|---|---|---|---|---|---|
| 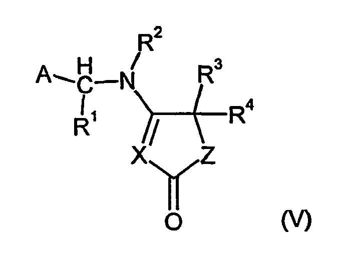 | | | | | | | | |
| Bsp. -Nr. | A | R¹ | R² | R³ | R⁴ | X | Z | Physikalische Daten^{a)} |
| V-3 | 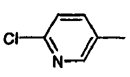 | -H | -Me | -H | -H | -CH= | -S- | 255 (MH⁺, 100); 3,10 min |
| V-4 | 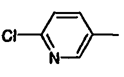 | -H | -Et | -H | -H | -CH= | -S- | 269 (MH⁺, 100); 3,61 min |
| V-5 | 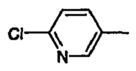 | -H | -Et | -H | -H | -N= | -CH₂- | 252 (MH⁺, 100); 1,65 min |
| V-6 | 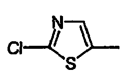 | -H | -Me | -H | -H | -N= | -CH₂- | 244 (MH⁺, 100); 1,39 min |
| V-7 | 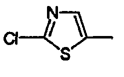 | -H | -Et | -H | -H | -N= | -CH₂- | 258 (MH⁺, 100); 2,16 min |

| | | | | | | | | |
|---|---|---|---|---|---|---|---|---|
| ^{a)} LC-MS (ESI-Positiv) (m/z, %); Rₜ (in min) (0,1 % HCOOH / Acetonitril) | | | | | | | | |

### Beispiel IX-1

### 6-Chlor-3-(N-(2,2-dimethyl-2H-azirin-3-yl)-N-methyl)-aminomethylpyridin

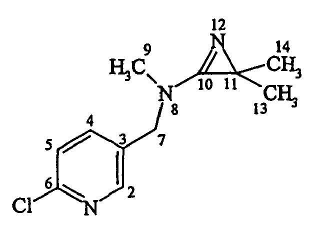

In einem sekurierten 250 ml Vierhalskolben mit Tropftrichter, Innenthermometer und Trockeneiskühler werden auf dem Eisbad 1.78 g (9.0 mmol) Diphosgen in 20 ml abs. CH₂Cl₂ vorgelegt. Bei 0-5 °C tropft man unter Stickstoff eine Lösung aus 3.63 g (15.0 mmol) N-(6-Chlor-3-pyridylmethyl)-N-methyl-2-methylpropanthioamid in 10 ml abs. CH₂Cl₂. Anschließend werden vier Tropfen abs. DMF zugegeben, das Reaktionsgemisch langsam auf Raumtemperatur gebracht und vier Stunden gerührt. Hiernach wird das überschüssige Phosgen und das Lösungsmittel im Wasserstrahlvakuum über eine Kühlfalle vollständig entfernt. Der zurückbleibende Feststoff wird unter Stickstoff in 60 ml abs. THF gelöst, auf 0-5 °C gebracht und unter kräftigem Rühren mit 1.68 g (15.0 mmol) DABCO versetzt. Das Reaktionsgemisch wird auf Raumtemperatur gebracht und 45 Minuten gerührt. Der entstandene voluminöse Niederschlag wird über ein Knie inert filtriert und zweimal mit abs. THF gewaschen. Die so erhaltene klare Lösung wird unter Stickstoff ohne weitere Aufarbeitung mit 1.95 g (30 mmol) festem Natriumazid versetzt und 48 Stunden gerührt. Zur Isolierung des Produktes werden die anorganischen Salze filtriert, das Lösungsmittel unter vermindertem Druck entfernt und der erhaltene Rückstand säulenchromatographisch mit Ethylacetat / Methanol =10 / 2 (R_{f}= 0.2) über Kieselgel eluiert.

| | | |
|---|---|---|
| Ausbeute | : 1.92 g (57 % d. Th.) | C₁₁H₁₄ClN₃ (M = 223.71 g / mol) |

MS (EI /70 eV)
m / z (%) = 224 (6), 222 (13), 178 (17), 128 (3), 126 (10), 99 (5), 97 (100), 70 (11), 68 (14), 57 (10), 56 (17), 44 (10), 43 (16), 42 (15), 41 (19), 40 (19), 39 (7).
¹H - NMR₃₀₀ (DMSO-d₆ / TMS) bei 373K
δ / ppm =1.22 (s, 6 H, H-13 u. H-14), 2.90 (s, 3 H, H-9), 4.44 (s, 2 H, H-7), 7.47 (dd, 1 H, H-5, *J =* 8.2 Hz, *J* < 1 Hz), 7.73 (ddt, 1 H, H-4, *J =* 8.2 Hz, *J =* 2.5 Hz, *J <* 1 Hz), 8.33 (dd, 1 H, H-2, *J*= 2.5 Hz, *J* < 1 Hz).

### Beispiel

### N-(6-Chlor-3-pyridylmethyl)-N-methyl-2-methylpropanthioamid

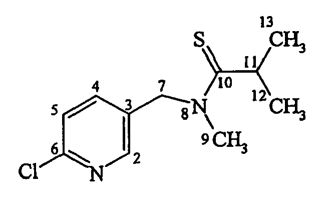

In einem 250 ml Zweihalskolben werden (30.0 mmol) *N*-(6-Chlor-3-pyridylmethyl)-*N*-methyl- 2-methylpropanamid (6.80 g) in 120 ml Benzol gelöst, mit 1.47 g (3.3 mmol) Phosphorpentasulfid versetzt und 1.5 Stunden zum Rückfluss erhitzt. Nach dieser Zeit wird auf Raumtemperatur abgekühlt, filtriert und das Filtrat unter vermindertem Druck am Rotationsverdampfer eingeengt. Zur Steigerung der Ausbeute sollte das Produkt noch am selben Tag säulenchromatographisch mit Dichlormethan / Diethylether 10 / 3 über Kieselgel eluiert werden; R_{f}= 0.8.

| | | |
|---|---|---|
| Smp. | : 56 °C | |
| Ausbeute | : 5.16 g (71 % d. Th.) | C₁₁H₁₅ClN₂S (M = 242.77 g / mol) |

MS (EI /70 eV)
m / z (%) = 245 (5), 244 (38), 243 (16), 242 (100) [M⁺], 211 (15), 210 (6), 209 (51), 170 (7), 169 (4), 168 (10), 167 (8), 129 (6), 128 (23), 127 (18), 126 (67), 116 (32), 103 (8), 101 (5), 99 (13), 98 (28), 90 (13), 88 (16), 87 (56), 84 (22), 83 (39), 82 (13), 75 (12), 74 (16), 73 (13), 70 (22), 45 (14), 43 (14), 42 (44), 41 (13).

| | |
|---|---|
| ¹H - NMR₃₀₀ (CDCl₃ / TMS) | * = doppelter Signalsatz aufgrund Amidresonaz |

δ /ppm = 1.25 (d, 1.2 H, H-12 u. H-13, ³*J=* 6.5 Hz)*, 1.26 (d, 6 H, H-12 u. H-13, ³*J* = 6.5 Hz), 3.14 (sept, 0.2 H, H-11, ³*J*= 6.5 Hz)*, 3.21 (sept, 1 H, H-11, ³*J*= 6.5 Hz), 3.29 (s, 0.6 H, H-9), 3.47 (s, 3 H, H-9)*, 4.93 (s, 0.4 H, H-7)*, 5.37 (s, 2 H, H-7), 7.31 (d, 1 H, H-5, *J*= 8.2 Hz), 7.36 (d, 0.2 H, H-5, *J*= 8.2 Hz)*, 7.43 (dd, 0.2 H, H-4, *J*= 8.2 Hz, *J*= 2.5 Hz)*, 7.71 (dd, 1 H, H-4, *J*= 8.2 Hz, *J*= 2.5 Hz), 8.24 (d, 0.2 H, H-2, *J=* 2.5 Hz)*, 8.32 (d, 1 H, H-2, *J*= 2.4 Hz).

### Kalium-N-cyaniminomethylsulfanylrnethanthiolat (XI)

### a) Herstellung des Dikaliumsalzes der Cyanimidodithiokohlensäure

In einem 500 ml Vierhalskolben mit KPG-Rührer, Tropftrichter, Thermometer und Rückflusskühler werden unter Stickstoffatmosphäre 21,0 g (0,50 mol) Cyanamid in 40 ml Ethanol auf 0°C gebracht. Bei dieser Temperatur werden nacheinander 41,9 g (0,55 mol) Schwefelkohlenstoff und dann 56,1 g (1,00 mol) Kaliumhydroxid in 180 ml Ethanol so zugetropft, dass die Innentemperatur nicht 5°C übersteigt (ca. vier Stunden). Nach beendeter Zugabe wird das Kühlbad entfernt und über Nacht bei Raumtemperatur gerührt. Der entstandene Feststoff wird filtriert, mit Ethanol gewaschen und im Vakuum getrocknet. Das entstandene Salz ist hygroskopisch und sollte nur kurze Zeit beim filtrieren mit Luft/Luftfeuchtigkeit in Berührung kommen. Das Salz wird im nächsten Schritt als Rohprodukt eingesetzt. Die Ausbeute liegt bei 75-80 % der Theorie.

### b) Kalium-N-cyaniminomethylsulfanylmethanthiolat

77,7 g (0,40 mol) des Rohproduktes werden in einem Gemisch aus 270 ml Aceton und 300 ml Wasser gelöst und auf 0°C gebracht. Bei dieser Temperatur werden unter starkem Rühren 56,8 g (0,40 mol) Methyliodid in 150 ml Aceton langsam über drei Stunden zugetropft. Nach beendeter Zugabe wird das Reaktionsgemisch eine Stunde bei 0°C und drei Stunden bei Raumtemperatur weitergerührt. Am Rotationsverdampfer wird bis zur Trockne alle Lösemittel entfernt. Der Rückstand wird in 250 ml abs. Aceton aufgenommen und bis auf die Hälfte am Rotationsverdampfer eingeengt. Zur Kristallisation des entstandenen Kaliumiodids wird das Reaktionsgemisch über Nacht im Kühlschrank belassen. Der entstandene Feststoff wird filtriert und das Filtrat weiter fast bis zur Trockne eingeengt. In der Kälte fällt das gewünschte Produkt. Der letzte Vorgang zur vollständigen Entfernung des Kaliumiodids kann nun wiederholt werden. Die Ausbeute liegt bei 75-80 % der Theorie. Die analytischen Daten stimmen mit der Literatur überein.

### Anwendungsbeispiele

### Beispiel A

### Meloidogyne-Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Gefäße werden mit Sand, Wirkstofflösung, Meloidogyne incognita-Ei-Larvensuspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

**Tabelle A**

| pflanzenschädigende Nematoden **Meloidogyne-Test** | | |
|---|---|---|
| Wirkstoffe | Wirkstoffkonzentration in ppm | Abtötungsgrad in % nach 14 Tagen |
| 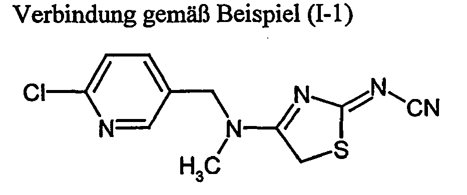 | 20 | 98 |

### Test mit Katzenflöhen / orale Aufnahme

| | |
|---|---|
| Testtiere: | Adulte von *Ctenocephalides felis* |
| Lösungsmittel: | Dimethylsulfoxid (DMSO) |

Zwecks Herstellung einer geeigneten Formulierung wird aus 20 mg Wirkstoff mit 1 ml DMSO eine geeignete Wirkstofflösung hergestellt. 20 µl dieser Formulierung werden zu 4 ml citriertem Rinderblut gegeben und verrührt.

20 nüchterne adulte Flöhe (*Ctenocephalides felis,* Stamm "Georgi") werden in eine Kammer(Ø 5 cm) eingesetzt, die oben und unten mit Gaze verschlossen ist. Auf die Kammer wird ein Metallzylinder gestellt, dessen Unterseite mit Parafilm verschlossen ist. Der Zylinder enthält die 4 ml Blut-Wirkstofformulierung, die von den Flöhen durch die Parafilmmembran aufgenommen werden kann. Während das Blut auf 37°C erwärmt wird, wird im Bereich der Flohkammern eine Temperatur von 25°C eingestellt. Kontrollen werden mit dem gleichen Volumen DMSO ohne Zusatz einer Verbindung vermischt. Es werden Dreifach-Bestimmungen durchgeführt.

Nach 24h wird die Mortalität in % bestimmt.

Verbindung die innerhalb von 24h eine mindestens 25 %ige Abtötung der Flöhe erzielen werden als wirksam beurteilt.

Bei diesem Test zeigen z.B. die folgenden Verbindungen der Herstellungsbeispiele eine Wirkung:

| | % Wirkung (Larvenmortalität) |
|---|---|
| Verbindung | 100 ppm |
| I-1 | 30 |

## Patentansprüche

1. Verbindungen der Formel (I) 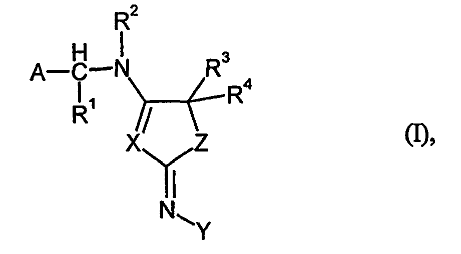 in welcher
A für jeweils gegebenenfalls substituiertes Aryl oder Hetaryl oder Heterocyclyl steht,
R¹ für Wasserstoff oder C₁-C₃-Alkyl steht,
R² für Wasserstoff oder C₁-C₃-Alkyl steht,
R³ für Wasserstoff oder C₁-C₃-Alkyl steht,
R⁴ für Wasserstoff oder C₁-C₃-Alltyl steht,
X für N oder CH steht,
Y für CN oder NO₂ steht,
Z für CH₂, O, S, SO, SO₂ oder NR⁵ steht und
R⁵ für Wasserstoff oder C₁-C₃-Alkyl steht.

2. Verbindungen der Formel (I) gemäß Anspruch 1, worin
A für gegebenenfalls durch Halogen, Cyano, Nitro, C₁-C₄-Alkyl, C₁-C₄-Halogenalkyl, C₁-C₄-Alkoxy oder C₁-C₄-Halogenalkoxy substituiertes Phenyl steht oder
A für Pyrazolyl, 1,2,4-Triazolyl, Oxazolyl, Isoxazolyl, Thiazolyl, Isothiazolyl, 1,2,5-Thiadiazolyl, Pyridyl, Pyrazinyl oder Pyrimidinyl steht, welche gegebenenfalls durch Fluor, Chlor, Brom, Cyano, Nitro, C₁-C₂-Alkyl (welches gegebenenfalls durch Fluor und/oder Chlor substituiert ist), C₁-C₂-Alkoxy (welches gegebenenfalls durch Fluor und/oder Chlor substituiert ist), C₁-C₂-Alkylthio (welches gegebenenfalls durch Fluor und/oder Chlor substituiert ist) oder C₁-C₂-Alkylsulfonyl (welches gegebenenfalls durch Fluor und/oder Chlor substituiert ist) substituiert sind, oder
A für einen gegebenenfalls durch Halogen oder C₁-C₃-Alkyl substituierten gesättigten C₅-C₆-Cycloalkylrest steht, in welchem eine Methylengruppe durch O oder S ersetzt ist,
R¹ für Wasserstoff, Methyl, Ethyl, n-Propyl oder i-Propyl steht,
R² für Wasserstoff, Methyl, Ethyl, n-Propyl oder i-Propyl steht,
R³ für Wasserstoff, Methyl, Ethyl, n-Propyl oder i-Propyl steht,
R⁴ für Wasserstoff, Methyl, Ethyl, n-Propyl oder i-Propyl steht,
X für N oder CH steht,
Y für CN oder NO₂ steht,
Z für CH₂, O, S oder NR⁵ steht und
R⁵ für Wasserstoff, Methyl, Ethyl, n-Propyl oder i-Propyl steht.

3. Verbindungen der Formel (I) gemäß Anspruch 1, worin
A für Thiazolyl oder Pyridyl steht, welche jeweils gegebenenfalls substituiert sind durch Halogen (insbesondere Chlor) oder C₁-C₃-Alkyl (insbesondere Methyl) oder
A für einen gegebenenfalls durch Halogen (insbesondere Chlor) oder C₁-C₃-Alkyl (insbesondere Methyl) substituierten Tetrahydrofurylrest steht,
R¹ für Wasserstoff oder Methyl steht,
R² für oder Methyl oder Ethyl steht,
R³ für Wasserstoff oder Methyl steht,
R⁴ für Wasserstoff oder Methyl steht,
X für N oder CH steht,
Y für CN oder NO₂ steht,
Z für CH₂, O, S oder NR⁵ steht, und
R⁵ für Wasserstoff oder Methyl steht.

4. Verbindungen der Formel (I) gemäß Anspruch 1, worin
A für einen der Reste 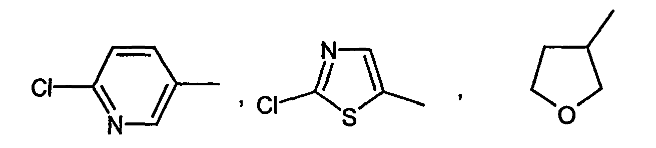 steht,
R¹ für Wasserstoff oder Methyl steht,
R² für Methyl oder Ethyl steht,
R³ für Wasserstoff oder Methyl steht,
R⁴ für Wasserstoff oder Methyl steht,
X für N oder CH steht,
Y für CN oder NO₂ steht,
Z für CH₂, O, S oder NR⁵ steht und
R⁵ für Wasserstoff oder Methyl steht.

5. Verfahren zur Herstellung von Verbindungen der Formel (I), **dadurch gekennzeichnet, dass** man Verbindungen der Formel (II) 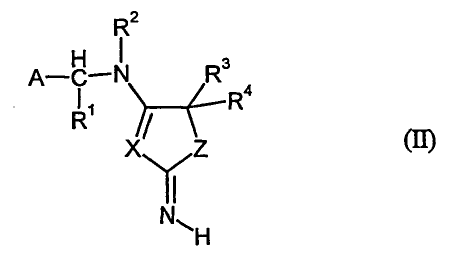 mit geeigneten Cyanierungsreagenzien oder mit geeigneten Nitrierungsreagenzien umsetzt.

6. Mittel zur Bekämpfung tierischer Schädlinge, **dadurch gekennzeichnet, dass** sie mindestens eine Verbindung der Formel (I) gemäß Anspruch 1 enthalten.

7. Verwendung von Verbindungen der Formel (I) gemäß Anspruch 1 zur Bekämpfung tierischer Schädlinge.

8. Herstellung von Mitteln zur Bekämpfung tierischer Schädlinge, **dadurch gekennzeichnet, dass** man Verbindungen der Formel (I) gemäß Anspruch 1 mit Verdünnungsmitteln und/oder oberflächenaktiven Stoffen vermischt.

## Claims

1. Compounds of the formula (I) 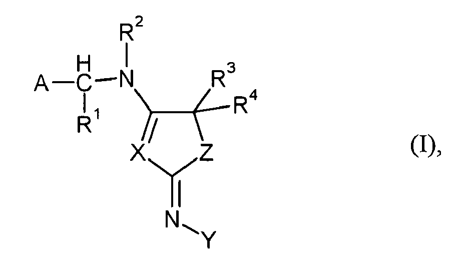 in which
A represents in each case optionally substituted aryl or hetaryl or heterocyclyl,
R¹ represents hydrogen or C₁-C₃-alkyl,
R² represents hydrogen or C₁-C₃-alkyl,
R³ represents hydrogen or C₁-C₃-alkyl,
R⁴ represents hydrogen or C₁-C₃-alkyl,
X represents N or CH,
Y represents CN or NO₂
Z represents CH₂, O, S, SO, SO₂ or NR⁵ and
R⁵ represents hydrogen or C₁-C₃-alkyl.

2. Compounds of the formula (I) according to Claim 1, in which
A represents optionally halogen-, cyano-, nitro-, C₁-C₄-alkyl-, C₁-C₄-haloalkyl-, C₁-C₄-alkoxy- or C₁-C₄-haloalkoxy-substituted phenyl or
A represents pyrazolyl, 1,2,4-triazolyl, oxazolyl, isoxazolyl, thiazolyl, isothiazolyl, 1,2,5-thiadiazolyl, pyridyl, pyrazinyl or pyrimidinyl, which are optionally substituted by fluorine, chlorine, bromine, cyano, nitro, C₁-C₂-alkyl (which is optionally substituted by fluorine and/or chlorine), C₁-C₂-alkoxy (which is optionally substituted by fluorine and/or chlorine), C₁-C₂-alkylthio (which is optionally substituted by fluorine and/or chlorine) or C₁-C₂-alkylsulfonyl (which is optionally substituted by fluorine and/or chlorine), or
A represents an optionally halogen- or C₁-C₃-alkyl-substituted saturated C₅-C₆-cycloalkyl radical in which one methylene group is replaced by O or S,
R¹ represents hydrogen, methyl, ethyl, n-propyl or i-propyl,
R² represents hydrogen, methyl, ethyl, n-propyl or i-propyl,
R³ represents hydrogen, methyl, ethyl, n-propyl or i-propyl,
R⁴ represents hydrogen, methyl, ethyl, n-propyl or i-propyl,
X represents N or CH,
Y represents CN or NO₂,
Z represents CH₂, O, S or NR⁵ and
R⁵ represents hydrogen, methyl, ethyl, n-propyl or i-propyl.

3. Compounds of the formula (I) according to Claim 1, in which
A represents thiazolyl or pyridyl, which are each optionally substituted by halogen (in particular chlorine) or C₁-C₃-alkyl (in particular methyl) or
A represents an optionally halogen- (in particular chlorine-) or C₁-C₃-alkyl- (in particular methyl-) substituted tetrahydrofuryl radical,
R¹ represents hydrogen or methyl,
R² represents hydrogen or methyl or ethyl,
R³ represents hydrogen or methyl,
R⁴ represents hydrogen or methyl,
X represents N or CH,
Y represents CN or NO₂,
Z represents CH₂, O, S or NR⁵, and
R⁵ represents hydrogen or methyl.

4. Compounds of the formula (I) according to Claim 1, in which
A represents one of the radicals 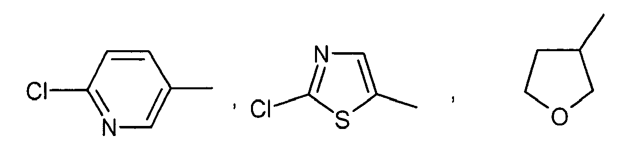
R¹ represents hydrogen or methyl,
R² represents methyl or ethyl,
R³ represents hydrogen or methyl,
R⁴ represents hydrogen or methyl,
X represents N or CH,
Y represents CN or NO₂,
Z represents CH₂, O, S or NR⁵ and
R⁵ represents hydrogen or methyl.

5. Process for preparing compounds of the formula (I), **characterized in that** compounds of the formula (II) 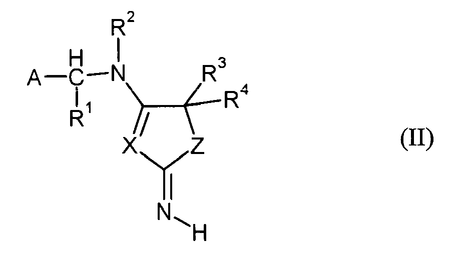 are reacted with suitable cyanating agents or with suitable nitrating agents.

6. Compositions for controlling animal pests, **characterized in that** they comprise at least one compound of the formula (I) according to Claim 1.

7. Use of compounds of the formula (I) according to Claim 1 for controlling animal pests.

8. Preparation of compositions for controlling animal pests, **characterized in that** compounds of the formula (I) according to Claim 1 are mixed with diluents and/or surfactants.

## Revendications

1. Composés de formule (I) 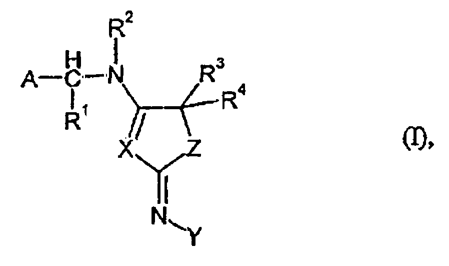 dans laquelle
A représente, dans chaque cas, un groupe aryle ou un groupe hétaryle ou un groupe hétérocyclyle, éventuellement substitué,
R¹ représente un atome d'hydrogène ou un groupe alkyle en C₁-C₃,
R² représente un atome d'hydrogène ou un groupe alkyle en C₁-C₃,
R³ représente un atome d'hydrogène ou un groupe alkyle en C₁-C₃,
R⁴ représente un atome d'hydrogène ou un groupe alkyle en C₁-C₃,
X représente un atome d'azote ou un groupe CH,
Y représente un groupe CN ou un groupe NO₂,
Z représente un groupe CH₂, un atome d'oxygène, un atome de soufre, un groupe SO, un groupe SO₂ ou un groupe NR⁵, et
R⁵ représente un atome d'hydrogène ou un groupe alkyle en C₁-C₃.

2. Composés de formule (I) selon la revendication 1, dans laquelle
A représente un groupe phényle éventuellement halogéno-, cyano-, nitro-, C₁-C₄-alkyl-, C₁-C₄-halogénoalkyl-, C₁-C₄-alcoxy- ou C₁-C₄-halogénoalcoxy-substitué, ou
A représente un groupe pyrazolyle, un groupe 1,2,4-triazolyle, un groupe oxazolyle, un groupe isoxazolyle, un groupe thiazolyle, un groupe isothiazolyle, un groupe 1,2,5-thiadiazolyle, un groupe pyridyle, un groupe pyrazinyle ou un groupe pyrimidinyle, qui sont éventuellement substitués par un atome de fluor, un atome de chlore, un atome de brome, un groupe cyano, un groupe nitro, un groupe alkyle en C₁-C₂ (qui est éventuellement substitué par un atome de fluor et/ou un atome de chlore), un groupe alcoxy en C₁-C₂ (qui est éventuellement substitué par un atome de fluor et/ou un atome de chlore), un groupe alkylthio en C₁-C₂ (qui est éventuellement substitué par un atome de fluor et/ou un atome de chlore) ou un groupe C₁-C₂-alkylsulfonyle (qui est éventuellement substitué par un atome de fluor et/ou un atome de chlore), ou
A représente un radical cycloalkyle en C₅-C₆ saturé, halogéno- ou C₁-C₃-alkyl-substitué, dans lequel un groupe méthylène est remplacé par un atome d'oxygène ou un atome de soufre,
R¹ représente un atome d'hydrogène, un groupe méthyle, un groupe éthyle, un groupe n-propyle ou un groupe isopropyle,
R² représente un atome d'hydrogène, un groupe méthyle, un groupe éthyle, un groupe n-propyle ou un groupe isopropyle,
R³ représente un atome d'hydrogène, un groupe méthyle, un groupe éthyle, un groupe n-propyle ou un groupe isopropyle,
R⁴ représente un atome d'hydrogène, un groupe méthyle, un groupe éthyle, un groupe n-propyle ou un groupe isopropyle,
X représente un atome d'azote ou un groupe CH,
Y représente un groupe CN ou un groupe NO₂,
Z représente un groupe CH₂, un atome d'oxygène, un atome de soufre ou un groupe NR⁵, et
R⁵ représente un atome d'hydrogène, un groupe méthyle, un groupe éthyle, un groupe n-propyle ou un groupe isopropyle.

3. Composés de formule (I) selon la revendication 1, dans laquelle
A représente un groupe thiazolyle ou un groupe pyridyle, qui sont chacun éventuellement substitués par un atome halogène (en particulier un atome de chlore) ou un groupe alkyle en C₁-C₃ (en particulier un groupe méthyle), ou
A représente un radical tétrahydrofuryle éventuellement halogéno- (en particulier chloro-) ou C₁-C₃-alkyl- (en particulier méthyl-) substitué,
R¹ représente un atome d'hydrogène ou un groupe méthyle,
R² représente un atome d'hydrogène ou un groupe méthyle ou un groupe éthyle,
R³ représente un atome d'hydrogène ou un groupe méthyle,
R⁴ représente un atome d'hydrogène ou un groupe méthyle,
X représente un atome d'azote ou un groupe CH,
Y représente un groupe CN ou un groupe NO₂,
Z représente un groupe CH₂, un atome d'oxygène, un atome de soufre ou un groupe NR⁵, et
R⁵ représente un atome d'hydrogène ou un groupe méthyle.

4. Composés de formule (I) selon la revendication 1, dans laquelle
A représente l'un des radicaux 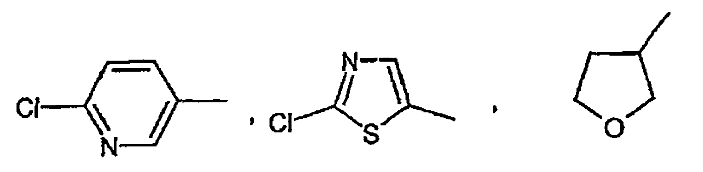
R¹ représente un atome d'hydrogène ou un groupe méthyle,
R² représente un groupe méthyle ou un groupe éthyle,
R³ représente un atome d'hydrogène ou un groupe méthyle,
R⁴ représente un atome d'hydrogène ou un groupe méthyle,
X représente un atome d'azote ou un groupe CH,
Y représente un groupe CN ou un groupe NO₂,
Z représente un groupe CH₂, un atome d'oxygène, un atome de soufre ou un groupe NR⁵, et
R⁵ représente un atome d'hydrogène ou un groupe méthyle.

5. Procédé de préparation de composés de formule (I), **caractérisé en ce que** des composés de formule (II) 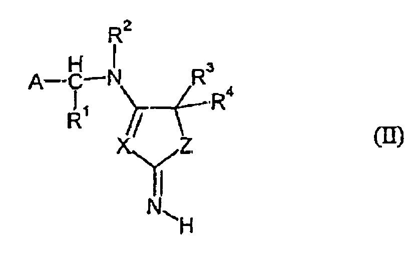 sont mis à réagir avec des agents de cyanation appropriés ou avec des agents de nitration appropriés.

6. Compositions destinées à lutter contre des animaux nuisibles, **caractérisées en ce qu'**elles comprennent au moins un composé de formule (I) selon la revendication 1.

7. Utilisation de composés de formule (I) selon la revendication 1 pour la lutte contre des animaux nuisibles.

8. Préparation de compositions destinées à lutter contre des animaux nuisibles, **caractérisée en ce que** des composés de formule (I) selon la revendication 1 sont mélangés avec des diluants et/ou des tensioactifs.
